(19) Europäisches Patentamt
European Patent Office
Office européen des brevets

(11) EP 4 737 456 A1

(12) **EUROPEAN PATENT APPLICATION**
published in accordance with Art. 153(4) EPC

(43) Date of publication:
**06.05.2026 Bulletin 2026/19**

(21) Application number: **24831118.5**

(22) Date of filing: **26.06.2024**

(51) International Patent Classification (IPC):
***C07D 471/04*** *(2006.01)* ***A61K 31/437*** *(2006.01)*
***A61P 29/00*** *(2006.01)*

(52) Cooperative Patent Classification (CPC):
**A61P 29/00; A61K 31/437; A61K 31/444; A61K 45/06; A61P 11/00; C07D 471/04; C07D 519/00**

(86) International application number:
**PCT/ES2024/070403**

(87) International publication number:
**WO 2025/003545 (02.01.2025 Gazette 2025/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.06.2023 ES 202330530**

(71) Applicants:
- **Universitat de València**
  **46010 Valencia (ES)**
- **Consejo Superior de Investigaciones Científicas (CSIC)**
  **28006 Madrid (ES)**
- **Universidad Complutense de Madrid (UCM)**
  **28040 Madrid (ES)**
- **Centro Nacional de Investigaciones Cardiovasculares Carlos III (F.S.P.)**
  **28029 Madrid (ES)**
- **IMHOTECH LABORATORIES, S.L.**
  **28221 Majadahonda Madrid (ES)**

(72) Inventors:
- **MARCILLA DÍAZ, Antonio**
  **46010 Valencia (ES)**
- **DEL POZO LOSADA, Carlos**
  **46010 Valencia (ES)**
- **SÁNCHEZ LÓPEZ, Christian Miquel**
  **46010 Valencia (ES)**
- **SÁNCHEZ SANCHO, Francisco Antonio**
  **28006 Madrid (ES)**
- **GARCÍA CSÁKY, Aurelio**
  **28040 Madrid (ES)**
- **OLIVER PÉREZ, Eduardo**
  **28029 Madrid (ES)**
- **IBÁÑEZ CABEZA. Borja**
  **28029 Madrid (ES)**
- **BAQUERO GÁLVEZ, María Gracia**
  **28006 Madrid (ES)**
- **ROSCALES GARCÍA, Silvia**
  **28040 Madrid (ES)**
- **CUMELLA MONTÁNCHEZ, José María**
  **28006 Madrid (ES)**
- **DEL POZO LOSADA, Jesús**
  **15004 A Coruña (ES)**

(74) Representative: **Cueto, Sénida**
**SP3 Patents S.L.**
**Los Madroños, 23**
**28891 Velilla de San Antonio (ES)**

# (54) DERIVATIVES OF IMIDAZO[1,2-A]PYRIDINE WITH ANTI-INFLAMMATORY ACTIVITY

(57) The present invention relates to the chemical synthesis and production of compounds derived from Imidazo[1,2-a]pyridine of formula I

EP 4 737 456 A1

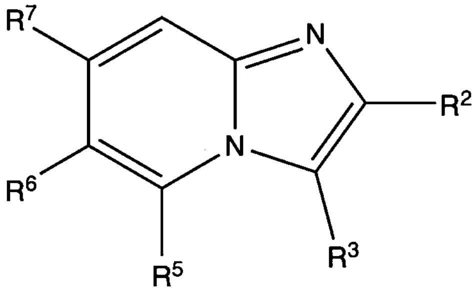

where $R^2$, $R^3$, $R^5$, $R^6$, and $R^7$ have the meanings indicated in the description, method of obtaining them, as well as their therapeutic use as a pharmaceutical composition for in vitro and in vivo anti-inflammatory use in diseases involving inflammatory and pro-fibrotic processes.

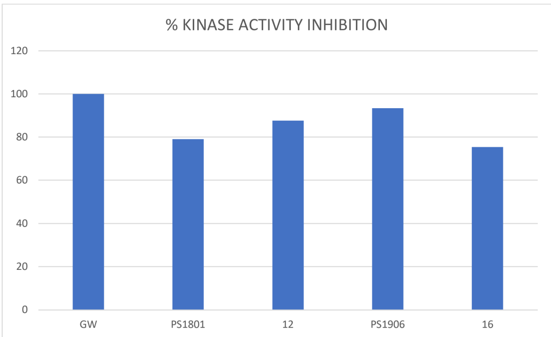


Figure 1

## Description

### FIELD OF THE INVENTION

The present invention relates to the production of synthetic chemicals for cosmetic, nutraceutical and therapeutic applications (Biomedical sector).

It falls within the field of preparing new compounds through the chemical synthesis of imidazo[1,2-a]pyridine derivatives using Suzuki coupling and heterocyclisation reactions, with potent anti-inflammatory activity *in vitro* in cell cultures and *in vivo* in mouse models.

### BACKGROUND OF THE INVENTION

Inflammation is a normal response of the immune system to infection. However, when allowed to continue unchecked, it can result in autoimmune or autoinflammatory disorders, neurodegenerative diseases, or even cancer. The growing understanding that chronic inflammation is crucial in many diseases opens up new ways for treatment.

A variety of safe and effective anti-inflammatory agents are currently available, including aspirin and other non-steroidal anti-inflammatory drugs (NSAIDs), with many more drugs in development. An effective anti-inflammatory drug should be able to inhibit the development of inflammation without interfering with normal homeostasis. Anti-inflammatory drugs typically inhibit cyclooxygenases (COX), which are involved in the biosynthesis of prostaglandins that promote inflammation. Conventional NSAIDs are associated with gastric and renal side effects, as they inhibit both constitutive COX-1 and inducible COX-2. Most selective COX-2 inhibitors (COXIBs) lack gastric side effects but are associated with cardiac side effects with long-term use.

On the other hand, excess reactive oxygen species (ROS) in the body cause oxidative stress, which damages the p53 protein and poly (ADP-ribosyl)ation (PARP), which, in turn, triggers the activation of proteases (calpains), protein degradation, and disruption of mitochondrial functions, which reduces ATP levels and ultimately leads to cell necrosis. ROS dysregulation can cause cellular dysfunction that results in the development of many diseases such as cancers, cardiovascular diseases, strokes, and ultimately death.

Current approaches to overcoming inflammation include the use of immuno-selective anti-inflammatory derivatives, selective glucocorticoid receptor agonists, statins, histone deacetylase inhibitors, PPAR agonists, resolvins and proteins, and TNF$\alpha$ inhibitors, among others.

Transforming growth factor-beta (TGF$\beta$) is a crucial agent in immune system homeostasis, involved, among other things, in inflammatory cell migration, growth inhibition, invasion, epithelial-mesenchymal transition (EMT), extracellular matrix (ECM) remodelling, and immunosuppression. However, although it is normally dynamically regulated and involved in maintaining tissue homeostasis, TGF$\beta$ is often chronically overexpressed in different pathologies, such as cancer, fibrosis, and inflammation. The TGF$\beta$ signalling pathway has become a popular target for drug development.

Furthermore, TGF$\beta$ is the most potent profibrogenic cytokine, and its expression is increased in almost all fibrotic diseases. There is growing evidence that reactive oxygen species (ROS) modulate TGF$\beta$ signalling through different pathways. In particular, TGF$\beta$1 increases ROS production and suppresses antioxidant enzymes, leading to a redox imbalance. ROS, in turn, induce/activate TGF$\beta$1 and mediate many of the fibrogenic effects of TGF$\beta$.

In this regard, compounds that selectively inhibit TGF$\beta$ receptors could have the potential to inhibit the inflammatory response and be developed for therapeutic applications in the treatment of fibrosis, late-stage carcinogenesis, atherosclerosis, and excessive scarring diseases in which TGF$\beta$ activity has been implicated in the signalling pathway. Inhibition of TGF$\beta$ is capable of reducing perivascular inflammation, thereby slowing disease progression. In this regard, deficiency of its receptor ALK5 is capable of inhibiting macrophage-mediated inflammation. In turn, the TGF$\beta$/ALK5 pathway has been proposed as a therapeutic target capable of restoring sensitivity to the anti-inflammatory activity of corticosteroids in inflammation caused by respiratory viral infections.

In most cells, TGF$\beta$ sends signals through the combination of TGF$\beta$R-II and ALK5.

ALK5 is a tyrosine kinase that is the type I receptor for tumour growth factor beta (TGF$\beta$-I) and has been described as a mediator in the inflammatory response, an inducer of fibrosis of various origins, and a promoter of cell survival, migration, differentiation and proliferation, related to various pathologies, including tumour processes. In fact, Alk5 inhibition has a clear inhibitory effect on the inflammatory response, promoting the anti-inflammatory response in human macrophages. Alk5 inhibition is referred to in Ling LE, Lee WC. Tgf-beta type I receptor (Alk5) kinase inhibitors in oncology. Curr Pharm Biotechnol. 2011 Dec;12(12):2190-202. https://doi.org/10.2174/138920111798808257.

On the other hand, it has recently been reported that targeting the TGF$\beta$/Smad signalling pathway attenuates the formation of keloid and hypertrophic scars. The formation of abnormal scars, including keloid and hypertrophic scars, is associated with a pathological disorganised healing process with chronic inflammation. The TGF$\beta$/Smad signalling pathway is the most canonical pathway through which collagen formation in fibroblasts and myofibroblasts is regulated. In recent years, several therapeutic strategies have been developed that target the TGF-$\beta$/Smad signalling pathway to

attenuate abnormal scar formation in the skin.

Very recently, the possible treatment of COVID-19 with TGFβ blockade has also been described (Chen et al., 2020; Ferreira-Gomes et al., 2021). The main cause of death in infected patients is severe acute respiratory distress syndrome (ARDS) caused by the SARS-Cov-2 virus. The syndrome is attributed to largely uncontrolled inflammatory responses characterised by a "cytokine storm", oedema and fibrosis in the lungs in the final stages. This leads to functional failure of the lungs and death of patients.

Acute respiratory distress syndrome (ARDS) is a form of non-cardiogenic pulmonaty oedema caused by alveolar damage secondary to intense inflammation of the lungs. Despite its high incidence, ARDS has no specific treatment beyond addressing the cause (antibiotics in the case of bacterial infection), minimising potentially harmful ventilation, and avoiding positive fluid balance.

Fibrosis in the lung may be primarily due to transforming growth factor beta (TGFβ). In addition, TGFβ also participates in fluid homeostasis in the lung. It has been proposed that the cytokine storm and pathogenesis of COVID-19 are a consequence of an unbalanced cytokine network resulting from increased biological activity of transforming growth factor-β (TGFβ) (Shen et al., 2021). The sudden and uncontrolled increase in active TGF-β (possibly with the help of some proinflammatory cytokines such as TNFα, IL-6, and IL-1β) inevitably results in rapid and massive oedema and fibrosis that remodel and ultimately block the airways. In addition, TGFβ, through internalisation of the epithelial sodium channel (ENaC), also suppresses the antioxidant system, and reactive oxygen species play an important role in lung injury, which is elevated during SARS-CoV-2 infection. Given all these characteristics, a possible immunotherapy for severe COVID-19 is suggested by blocking transforming growth factor beta (TGFβ).

The anti-inflammatory evaluation of imidazo[1,2-a]pyridinecarboxylic acid derivatives (Márquez-Flores et al, 2012), 2,3,6-substituted imidazo[1,2-a]pyridine derivatives with anti-inflammatory activity in vitro activity measured by the bovine serum albumin (BSA) protein denaturation assay (Budumuru et al., 2019), and 7-aryl-imidazo[1,2-a]pyridin-3-ylquinolines as ALK inhibitors (Engers et al., 2020) have been described.

Galunisertib and Vactosertib are TGFβ and/or ALK5 pathway inhibitors that have demonstrated a favourable safety profile in Phase I trials and are being considered as potential antitumour agents due to the involvement of the aforementioned pathway in cell proliferation and migration.

WO 2013147711 relates to compounds, including certain imidazo-pyridines, which act as inhibitors of MNK2a, MNK2b, MNK1a and MNK1b kinases, which interact with MAP kinase, to pharmaceutical compositions comprising these compounds, and to the use of the compounds for the preparation of a medicament for the prophylaxis and treatment of diseases, such as proliferative diseases, e.g. cancer, inflammatory diseases, Alzheimer's disease. The compounds disclosed by WO2013147711 are different from those prepared in the present invention. Furthermore, there is no evidence in WO2013147711 showing that said compounds have inhibitory capacity on tyrosine kinase Alk5, which is the receptor I of transforming growth factor or TGFβ, which is the basis of the activity of the compounds of the present invention.

WO2008014219 discloses a method for inhibiting the activity/function of PB kinases using thiazolidinedione derivatives. Among the disclosed compounds are certain imidazo-pyridines that act as inhibitors. They are said to be useful for treating selected pathological conditions such as autoimmune disorders, inflammatory diseases, or lung injuries. The compounds disclosed by WO2008014219 are different from those prepared in the present invention. Furthermore, there is no evidence in WO2008014219 showing that these compounds have the ability to inhibit tyrosine kinase Alk5, which is the receptor I of transforming growth factor or TGFβ, which is the basis of the activity of the compounds of the present invention.

US2004122044 discloses pharmaceutical formulations with compounds having an imidazo[1,2-a]-pyridin-3-ylamide skeleton or amine compounds as active ingredients for inhibiting nitric oxide synthase. Some treatments of diseases that can be treated with such compounds are also mentioned. However, the compounds disclosed by US2004122044 are different from those prepared in the present invention. Furthermore, it only shows examples of anti-NO synthase activity, but there is no evidence in US2004122044 showing that these compounds have activity for the specific diseases or problems for which the compounds of the present invention are intended.

WO2019099336 refers to indole-derived compounds, including certain imidazo-pyridines, useful in the treatment of autoimmune and inflammatory diseases. However, the imidazopyridines disclosed do not coincide in any case with the compounds of the present invention, since according to formula (I) of independent claim 1, when the substituent at positions 6 or 7 of the imidazopyridine skeleton is a heterocycle, said heterocycle is never substituted with a piperidine. Therefore, the activity in the field of medicine does not have to be the same, or even similar, to that of the compounds of the present invention. In particular, WO2019099336 does not demonstrate that the disclosed indoles have the ability to inhibit tyrosine kinase Alk5, which is the receptor I of transforming growth factor or TGFβ, which is the basis of the activity of the compounds of the present invention.

The article RODRIGUEZ, J. C. et al., "Microwave-assisted synthesis and luminescent activity of imidazo[1,2a] pyridine derivatives"; Journal of Heterocyclic Chemistry, 2020, discloses a series of compounds that are potentially useful in the field of medicine. These compounds are different from those obtained in the present invention, and this document does not demonstrate any medicinal or cosmetic activity for the compounds shown.

The compounds of the invention use the inhibition of ALK5, belonging to the TGFβ receptor family, as an anti-inflammatory strategy capable of preventing fibrotic events, among others (Nolte and Margadant, 2020). These compounds are useful in the treatment of cancer, lung diseases, fibrotic diseases, prevention of keloid and hypertrophic scar formation, and as a treatment for severe COVID-19.

The compounds of the invention have the chemical structure of a planar aromatic imidazo[1,2-a]pyridine skeleton functionalised at different positions of this heterocyclic skeleton, with particular emphasis on those derivatives substituted at C-7 with an aromatic heterocyclic system. Some of them were prepared from 7-bromoimidazo[1,2-a]pyridine and (hetero)arylboronic acids using Suzuki couplings. The imidazo[1,2-a]pyridine skeleton is known as a privileged structure as it is present in many commercial drugs such as Zolpidem, Olprinone, Soraprazan and many other compounds that are currently in different stages of biological testing and preclinical evaluation, showing multiple therapeutic applications.

The compounds of the invention are compounds based on structures with inhibitory activity against tyrosine kinase Alk5, which is the receptor I for transforming growth factor or TGFβ.

They exhibited potent anti-inflammatory activity both in vitro (on human macrophages in culture and human macrophages in culture) and in vivo in an animal model of acute lung injury, as well as an anti-fibrotic effect in a murine model.

**References:**

Chen et al., 2020: Chen W. A potential treatment of COVID-19 with TGF-β blockade. Int J Biol Sci. 2020 Apr 21; 16(11):1954-1955.
https://doi.org/10.7150/ijbs.46891

Ferreira-Gomes et al., 2021: Ferreira-Gomes M, Kruglov A, Durek P, Heinrich F, Tizian C, Heinz GA, Pascual-Reguant A, Du W, Mothes R, Fan C, Frischbutter S, Habenicht K, Budzinski L, Ninnemann J, Jani PK, Guerra GM, Lehmann K, Matz M, Ostendorf L, Heiberger L, Chang HD, Bauherr S, Maurer M, Schönrich G, Raftery M, Kallinich T, Mall MA, Angermair S, Treskatsch S, Dörner T, Corman VM, Diefenbach A, Volk HD, Elezkurtaj S, Winkler TH, Dong J, Hauser AE, Radbruch H, Witkowski M, Melchers F, Radbruch A, Mashreghi MF. SARS-CoV-2 in severe COVID-19 induces a TGF-β-dominated chronic immune response that does not target itself. Nat Commun. 2021 Mar 30;12(1):1961. https://doi.org/10.1038/s41467-021-22210-3.

Shen et al., 2021: Shen WX, Luo RC, Wang JQ, Chen ZS. Features of Cytokine Storm Identified by Distinguishing Clinical Manifestations in COVID-19. Front Public Health. 2021 May 24;9:671788. https://doi.org/10.3389/fpubh.2021.671788.

Márquez-Flores et al., 2012: Márquez-Flores YK, Campos-Aldrete ME, Sagado-Zamora H, Correa-Basurto J, Meléndez-Camargo ME. Acute and chronic anti-inflammatory evaluation of imidazo[1,2-A]pyridine carboxylic acid derivatives and docking analysis. Medicinal Chemistry Research, 2012; 3491-3498. https://doi.org/10.1007/s00044-011-9870-3.

Budumuru et al.; 2019: Budumuru P, Golagani S, and Pushpanjali B. Microwave-assisted synthesis of imidazo[1,2-a]pyridine derivatives and their anti-inflammatory activity IJPSR, 2019; 10(3): 1172-1179 http://dx.doi.org/10.13040/IJPSR.0975-8232.10(3).1172-79

Engers et al., 2020: Engers DW, Bollinger SR, Felts AS, Vadukoot AK, Williams CH, Blobaum AL, Lindsley CW, Hong CC, Hopkins CR. Discovery, synthesis and characterization of a series of 7-aryl-imidazo[1,2-a]pyridine-3-ylquinolines as activin-like kinase (ALK) inhibitors. Bioorg Med Chem Lett. 2020 Sep 15;30(18):127418. https://doi.org/10.1016/j.bmcl.2020.127418

Nolte and Margadant et al., 2020: Nolte M, Margadant C. Controlling Immunity and Inflammation through Integrin-Dependent Regulation of TGF-β. Trends Cell Biol. 2020 Jan;30(1):49-59. https://doi.org/10.1016/j.tcb.2019.10.002

**DESCRIPTION OF THE INVENTION**

The present invention relates to a compound of formula I:

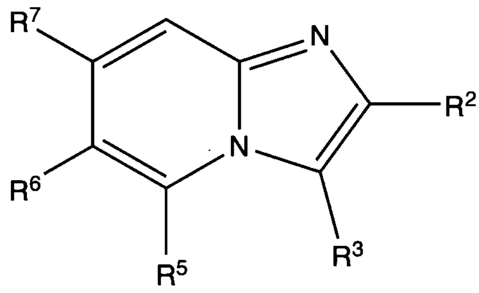

I

or a pharmaceutically acceptable salt thereof, wherein:

$R^2$ represents H; an aryl, where the aryl group may be unsubstituted or may be substituted with alkyl groups of 1 to 5 carbon atoms, or substituted with a haloalkyl (such as $-CF_3$), or substituted with an alkoxy group of 1 to 5 carbon atoms, such as methoxy, ethoxy, propyl, butanyl,

$R^3$ represents H, a halogen (F, Cl, Br, I); a haloalkyl; an alkoxycarbonyl of 2 to 5 carbon atoms; an amino; an aryl or heteroaryl, where the aryl and heteroaryl groups may be unsubstituted or may be substituted with alkyl groups of 1 to 5 carbon atoms or with one or more alkoxycarbonyl groups of 2 to 5 carbon atoms (such as ethoxycarbonyl),

$R^5$ represents H; an alkenyl of 2 to 10 carbon atoms; an alkoxycarbonyl of 2 to 5 carbon atoms; a nitro group; a carboxyl; an alkyl of 1 to 5 carbon atoms unsubstituted or substituted with one or more hydroxy groups, preferably substituted with one hydroxy group; by an N-(3-methylene-2-oxoindolin-5-yl)-3-(piperidin-1-yl)propanamide) group or a carbaldehyde,

$R^6$ represents a hydrogen atom (H); an unsubstituted alkyl group of 1 to 5 carbon atoms; an alkyl group of 1 to 5 carbon atoms substituted with one or more hydroxy groups, preferably substituted with one or more hydroxy groups, such as hydroxymethyl; an alkoxy group of 1 to 5 carbon atoms; halogen (F, Cl, Br, I); an alkoxycarbonyl group (-O-(C=O)-R) where R has 1 to 3 carbon atoms, such as methoxycarbonyl, ethoxycarbonyl, propoxycarbonyl; a nitro group; an alkenyl group of 2 to 10 atoms, such as 3, 4, 5, 6, 7, 8, 9 or 10 carbon atoms - preferably 3 to 10 carbon atoms, unsubstituted or substituted, for example, by an aryl group, such as phenyl; alkenyl of 2 carbon atoms substituted with phenyl (styryl); an aryl, where the aryl group may be unsubstituted or may be substituted by one or more hydroxyl groups (e.g., substituted by one hydroxyl), or substituted with one or more methoxyl groups; a heteroaryl, such as furanyl, thiophenyl, pyrrolyl or pyridinyl; or

a heteroaryl where the heteroaryl group is selected from dibenzothiophenyl, a group of formula II, a group of formula III:

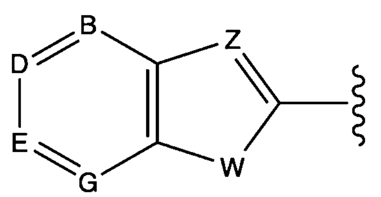

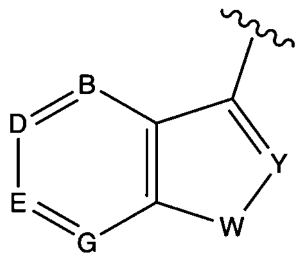

II

III

where D, B, E, G independently represent N, CH or C-R, where R may be an unsubstituted alkyl of one to three carbon atoms, an unsubstituted aryl or an unsubstituted cycloalkyl
Z represents CH or N
Y represents CH or N
W represents O or S or NH,

such as benzothiophenyl, benzofuranyl, benzopyrolyl,

$R^7$ represents a hydrogen atom (H); an unsubstituted aryl group or an aryl group substituted with one or more hydroxy groups, such as hydroxyphenyl (e.g., substituted by a hydroxyl), or substituted with one or more methoxy groups; a halogen (F, Cl, Br, I); an alkenyl of 2 to 10 atoms, such as 3, 4, 5, 6, 7, 8, 9 or 10 carbon atoms - preferably 3 to 10 carbon atoms unsubstituted or substituted by an aryl group such as phenyl; alkenyl of 2 carbon atoms substituted with a phenyl (styryl); an alkoxycarbonyl of 2 to 5 carbon atoms; a heteroaryl group, such as furanyl, thiophenyl, pyrrolyl or pyridinyl; or heteroaryl where the heteroaryl group is selected from benzothiophenyl, benzofuranyl, benzopyrolyl; dibenzothiophenyl, a group of formula II and a group of formula III as defined above,

- wherein
  - at least 3 of the substituents $R^2$, $R^3$, $R^5$, $R^6$ and $R^7$ are simultaneously H, and the position of carbon 8 of the imidazo[1,2a]pyridine structure has no substituents, and
  - when $R^5$ is a carbaldehyde, at least one of $R^2$, $R^3$, $R^6$ or $R^7$ is other than H,
  - when $R^6$ is an aryl or a substituted aryl, $R^3$ is not a substituted aryl
  - when $R^3$ represents an amino group, $R^6$ does not represent hydrogen
  - when $R^2$ represents an unsubstituted aryl, or an aryl substituted with a methyl or methoxy group, at least one of the substituents $R^2$, $R^3$, $R^5$, $R^6$ and $R^7$ is not H
  - at least one of the groups $R^3$, $R^5$, $R^6$, $R^7$ is other than H, when $R^2$ is an unsubstituted aryl, or is an aryl substituted with a halogen, a methyl or a methoxy.

or is a compound of formula (I) in which
$R^2$ represents an aryl substituted with an alkoxy of 1 to 5 carbon atoms and simultaneously $R^6$ is an aryl group substituted with a halogen (F, Cl, Br, I), and the other substituents have the meanings indicated above.

The group is N-(3-methylene-2-oxoindolin-5-yl)-3-(piperidin-1-yl)propanamide) at the $R^5$ position, resulting in a compound of formula I, which is as shown below:

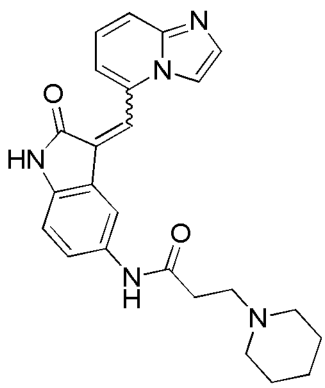

"Aryl" means a phenyl group; a condensed aromatic carbocycle, such as naphthyl or anthranyl; or indanyl, without heteroatoms.
"Alkyl", unless otherwise specified, means alkyl with 1 to 5 carbon atoms, in particular methyl, ethyl, propyl or butyl.
"Alkenyl", unless otherwise specified, means alkenyl with 2 to 10 carbon atoms.
"Heteroaryl", unless otherwise specified, means a monocyclic or fused bicyclic group composed of between 5 and 10 ring members, having at least one aromatic residue and containing 1 to 3 heteroatoms selected from oxygen, sulphur and nitrogen.
"Haloalkyl", unless otherwise indicated, means an alkyl group of 1 to 3 carbon atoms, substituted with one or more halogen atoms selected from fluorine, chlorine, bromine or iodine.
"Cycloalkyl" has the usual meaning, i.e. a univalent substituent derived from a cycloalkane without side chains.

According to particular embodiments, $R^2$ represents an aryl substituted with a $CF_3$ group or with an alkoxy group of 1 to 5 carbon atoms, for example, substituted with methoxy; and preferably for these embodiments, $R^3$ and $R^5$ are simultaneously H.

According to additional particular embodiments, $R^2$ represents an aryl substituted with a para-methoxyphenyl group, and preferably, in addition, $R^3$ and $R^5$ are simultaneously H.

According to additional specific embodiments, $R^2$ represents an aryl substituted with an alkoxy having 1 to 5 carbon atoms, for example substituted with a methoxy, and simultaneously $R^6$ is an alkyl having 1 to 5 carbon atoms, for example a methyl, or simultaneously $R^6$ is an aryl group substituted with a hydroxyl or with a halogen (F, Cl, Br, I); and preferably for these embodiments, $R^3$ and $R^5$ are simultaneously H.

According to additional particular embodiments, $R^2$ represents a group selected from an aryl substituted with alkoxy of 1 to 5 carbon atoms, for example, substituted with a methoxy; and simultaneously $R^6$ is an alkyl of one to five carbon atoms, for example a methyl, or simultaneously $R^6$ is an aryl group substituted with a hydroxyl or with a halogen; and preferably for these embodiments, $R^3$ and $R^5$ are simultaneously H.

According to additional particular embodiments, $R^2$ represents a phenyl substituted with an alkoxy of 1 to 5 carbon atoms, for example, substituted with p-methoxy, or $R^2$ represents phenyl substituted with trihalomethyl, such as a p-trifluoromethylphenyl group; and simultaneously $R^7$ is a heteroaryl group, for example, 2-benzo[b]furyl; and preferably, for these embodiments, $R^3$, $R^4$, $R^5$ and $R^6$ are simultaneously H.

According to additional specific embodiments:

- $R^2$ represents an aryl substituted with alkoxy of 1 to 5 carbon atoms, for example, substituted with a methoxy, and $R^7$ is an aryl substituted with hydroxyl or with an alkoxy group of 1 to 5 carbon atoms, for example, substituted with a methoxy, or

- $R^2$ represents an aryl substituted with alkoxy of 1 to 5 carbon atoms, for example, substituted with methoxy, and $R^7$ represents an alkenyl substituted with an aryl group, for example, substituted with a phenyl; and preferably for these embodiments, $R^3$ and $R^5$ are simultaneously H.

According to additional particular embodiments, $R^2$ represents an aryl substituted with an alkoxy of 1 to 5 carbon atoms, for example, substituted with methoxy, and $R^7$ is an aryl substituted with hydroxyl or with an alkoxy group of 1 to 5 carbon atoms, for example, substituted with a methoxy, or an alkenyl substituted with an aryl group, e.g., substituted with a phenyl; and preferably for these embodiments, $R^3$ and $R^5$ are simultaneously H.

According to preferred embodiments, $R^2$ represents an aryl substituted with an alkoxy of 1 to 5 carbon atoms, for example, substituted with a methoxy, $R^3$ is H, $R^6$ is H, an alkyl group or an aryl group, and $R^7$ is H or an aryl substituted with hydroxyl or with an alkoxy group of 1 to 5 carbon atoms, for example, substituted with a methoxy; and preferably, for these embodiments, $R^3$ and $R^5$ are simultaneously H.

According to additional particular embodiments, $R^3$ is an an unsubstituted aryl; and preferably for these embodiments, $R^2$, $R^5$ and $R^6$ are simultaneously H; and more preferably for these embodiments, $R^2$, $R^5$, $R^6$ and $R^7$ are simultaneously H.

According to additional particular embodiments, $R^3$ represents an aromatic heterocycle, which may be unsubstituted, or may be substituted with an alkoxycarbonyl group of 2 to 5 carbon atoms, such as an ethoxycarbonyl group; and preferably for these embodiments, $R^2$, $R^5$ and $R^6$ are simultaneously H; and more preferably for these embodiments, $R^2$, $R^5$, $R^6$ and $R^7$ are simultaneously H.

According to additional particular embodiments, $R^3$ is a group selected from benzofuranyl, benzothiophenyl and indole, which may be unsubstituted or may be substituted with an alkoxycarbonyl group of 2 to 5 carbon atoms, such as an ethoxycarbonyl group; and preferably for these embodiments, $R^2$, $R^6$ and $R^6$ are simultaneously H; and more preferably for these embodiments, $R^2$, $R^5$, $R^6$ and $R^7$ are simultaneously H.

According to additional particular embodiments, $R^3$ is a group selected from benzofuranyl, benzothiophenyl and indole, which may be unsubstituted or may be substituted with an alkoxycarbonyl group of 2 to 5 carbon atoms, such as an ethoxycarbonyl group; and simultaneously $R^7$ is a group selected from benzofuranyl, benzothiophenyl and indole; preferably for these embodiments, $R^2$, $R^6$ and $R^6$ are simultaneously H.

According to additional particular embodiments, $R^5$ represents H, an ethoxycarbonyl, a nitro group, a carboxyl, a hydroxymethyl, a carbaldehyde, or an ethoxyl, and preferably, for these embodiments, $R^2$, $R^3$, $R^6$, and $R^7$ are simultaneously H.

According to additional particular embodiments, $R^7$ and $R^6$ independently represent a fragment

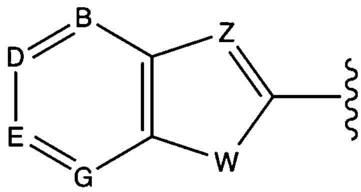

II

wherein D, B, E, G independently represent CH or C-R, where R may be unsubstituted alkyl of one to three carbon atoms, unsubstituted aryl or unsubstituted cycloalkyl, Z represents CH and W represents O.

According to additional particular embodiments, $R^7$ or $R^6$ represents a fragment.

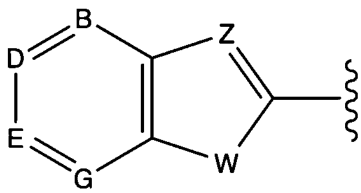

II

wherein D, B, E, G independently represent CH, Z represents CH, and W represents O.

According to additional particular embodiments, $R^6$ represents an alkyl group of 1 to 5 carbon atoms or alkoxycarbonyl (-O-(C=O)-R) where R has 1 to 3 carbon atoms, such as methoxycarbonyl; and preferably, for these embodiments, $R^2$, $R^3$, $R^5$ and $R^7$ are simultaneously H.

According to additional particular embodiments, $R^7$ represents an unsubstituted aryl group, such as phenyl, and preferably, for these embodiments, $R^2$, $R^3$, $R^5$, and $R^7$ are simultaneously H.

According to additional particular embodiments, $R^7$ represents a benzofuranyl group, and preferably, for these embodiments, $R^2$, $R^3$, $R^5$, and $R^3$ are simultaneously H.

According to particular embodiments, the compound of formula I is selected from:

- Imidazo[1,2-a]pyridine-5-carboxylic acid (2)
- Ethyl imidazo[1,2-a]pyridine-5-carboxylate (3)
- 3-Phenylimidazo[1,2-a]pyridine (4)
- Imidazo[1,2-a]pyridin-5-ylmethanol (5)
- 2-(4-Methoxyphenyl)-6-methylimidazo[1,2-a]pyridine (8)
- 4-(Imidazo[1,2-a]pyridin-7-yl)phenol (12)
- 4-(2-(4-(Trifluoromethyl)phenyl)imidazo[1,2-a]pyridin-7-yl)phenol (13)
- 4-(2-(4-(Trifluoromethyl)phenyl)imidazo[1,2-a]pyridin-6-yl)phenol (15)
- 4-(2-(4-Methoxyphenyl)imidazo[1,2-a]pyridin-7-yl)phenol (16)
- 4-(2-(4-Methoxyphenyl)imidazo[1,2-a]pyridin-6-yl)phenol (17)
- (E)-7-Styrylimidazo[1,2-a]pyridine (19)
- (E)-2-(4-Methoxyphenyl)-7-styrylimidazo[1,2-a]pyridine (20)

- 7-(3,4-Dimethoxyphenyl)imidazo[1,2-a]pyridine (21)
- 6-Iodo-2-(4-(trifluoromethyl)phenyl)imidazo[1,2-a]pyridine (25)
- 7-(Benzofuran-2-yl)imidazo[1,2-a]pyridine (26) PS1906
- 4-(Imidazo[1,2-a]pyridin-6-yl)phenol (27)
- (E)-6-Styrylimidazo[1,2-a]pyridine (28)
- 6-(Benzofuran-2-yl)imidazo[1,2-a]pyridine (29)
- 6-(3,4-Dimethoxyphenyl)imidazo[1,2-a]pyridine (30)
- Ethyl 5-(imidazo[1,2-a]pyridin-3-yl)-1H-indole-2-carboxylate (31) (PS1801)
- 7-(Benzofuran-3-yl)imidazo[1,2-a]pyridine (32)
- 6-(Benzofuran-3-yl)imidazo[1,2-a]pyridine (33)
- 7-(Furan-3-yl)imidazo[1,2-a]pyridine (34)
- 6-(Furan-3-yl)imidazo[1,2-a]pyridine (35)
- 7-(Thiophen-3-yl)imidazo[1,2-a]pyridine (36)
- 6-(Thiophen-3-yl)imidazo[1,2-a]pyridine (37)
- 7-(Pyridin-4-yl)imidazo[1,2-a]pyridine (38)
- 6-(Pyridin-4-yl)imidazo[1,2-a]pyridine (39)
- 7-(Dibenzothiophen-3-yl)imidazo[1,2-a]pyridine (40)
- 6-(Dibenzothiophene-3-yl)imidazo[1,2-a]pyridine (41)
- Ethyl 5-(7-(benzofuran-2-yl)-3-imidazo[1,2-a]pyridin)-1H-indole-2-carboxylate, (42)
- N-(3-(imidazo[1,2-a]pyridin-5-ylmethylen)-2-oxoindolin-5-yl)-3-(piperidin-1-yl)propanamide.

According to a preferred embodiment, the compound of formula I is 7-(benzofuran-2-yl)imidazo[1,2-a]pyridine. (26), PS1906.

According to an additional preferred embodiment, the compound of formula I is ethyl 5-(imidazo[1,2-a]pyridin-3-yl)-1H-indole-2-carboxylate, compound (31).

The present invention also relates to a process for preparing a compound of formula I comprising:

1) Reacting an aminopyridine of formula (IV) (commercially available)

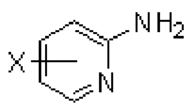

(IV)

wherein X is Cl, Br, or I, with R-$B(OH)_2$ wherein R is a fragment of formula $R^7$ or $R^6$ defined above to obtain a compound of formula (V)

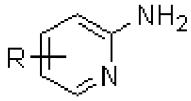

(V)

and

2) reacting the compound of formula (V) with a haloketone of formula (VI)

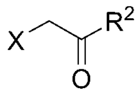

(VI)

wherein X is Cl, Br or I and $R^2$ giving rise to a compound of formula **I** defined above.

The reaction in step 1) can be carried out in the presence of a Pd(0) catalyst and an inorganic base such as potassium carbonate.

The temperature for the reaction in step 1) can be between 80 and 150 °C.

The reaction in step 1) can be carried out conventionally (18-24 h) or using a microwave (1-5 h).

The solvent for the reaction in step 1) can be, for example, a polar solvent such as 1,2-dimethoxyethane, 1,4-dioxane, DMF, DMSO.

The reaction in step 2) can be carried out in the presence of a solvent, such as those mentioned for step 1), and an organic or inorganic base, such as sodium or potassium bicarbonate, sodium or potassium phosphate, potassium acetate, or potassium methoxide.

The reaction in step 2) can be carried out at a temperature between 25 and 120 °C, preferably under reflux.

An additional alternative to the procedure for preparing a compound of formula I comprises:

1) Reacting an aminopyridine of formula (IV)

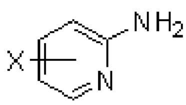

(IV)

wherein X is Cl, Br, or I, with a haloketone of formula (VI)

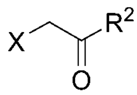

(VI)

wherein X is Cl, Br or I and $R^2$ has the meaning given above, resulting in a compound of formula VII

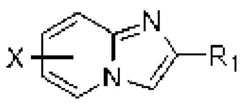

(VII

2) and reacting the compound of formula (VII) with R-B(OH)$_2$, wherein R- is a fragment of formula $R^6$ or $R^7$ defined above, yielding a compound of formula I.

In the case of compounds of formula I substituted at position 7 with an aromatic heterocyclic substituent, the process comprises reacting 7-bromoimidazo[1,2-a]pyridine and (hetero)arylboronic acids via Suzuki couplings.

A third alternative procedure for preparing compounds of formula I, when they carry a heteroaryl as a substituent on carbon C3, is to use the Heck reaction, which comprises reacting an imidazopyridine with a haloheteroaryl in the presence of a Pd(0) catalyst that is generated in situ.

To analyse the Alk5 inhibitory activity of the compounds of the invention in in vitro kinase activity assays, the studies by Roth et al. (2010) were followed. For this purpose, recombinant Alk5 (Abcam, ref. ab105908) was used, and

kinase activity was determined using the commercial Kinase-Glo® Luminiscent kinase assay platform (Promega, ref. TB372). The compound GW788388 Hydrate (Sigma-Aldrich ref. SML0116) was used as a positive inhibition control, whose formula is (4-{4-[3-(pyridin-2-yl)-1H-pyrazol-4-yl]-pyridin-2-yl}-N-(tetrahydro-2H-pyran-4-yl)benzamide hydrate, a potent inhibitor of Alk5 for oral use.

The invention has applications in biomedicine, in the pharmaceutical sector, as well as in cosmetics and nutraceuticals. Effective formulations can be obtained for topical, oral and parenteral application.

An additional object of the invention relates to a compound of formula I, as described above, for use as a medicament.

According to particular embodiments, such use comprises the treatment of an inflammatory disease in a subject.

The terms 'treatment' and 'treat' used herein refer to the medical treatment of a subject with the intention of curing, improving, stabilising, or preventing a disease, pathological condition, or disorder. This term includes active treatment, i.e., treatment specifically aimed at improving a disease, pathological condition or disorder, and also includes causal treatment, i.e., treatment aimed at eliminating the cause of the associated disease, pathological condition or disorder. In addition, this term includes palliative treatment, i.e., treatment designed to alleviate symptoms rather than cure the disease, pathological condition or disorder; preventive treatment, i.e. treatment aimed at minimising or partially or completely inhibiting the development of the associated disease, pathological condition or disorder; and supportive treatment, i.e. treatment used to complement other specific therapy aimed at improving the associated disease, pathological condition or disorder. It is understood that treatment, even if intended to cure, improve, stabilise or prevent a disease, pathological condition or disorder, does not necessarily actually produce cure, improvement, stabilisation or prevention. The effects of treatment may be measured or evaluated as described herein and as known in the art, as appropriate for the disease, pathological condition or disorder in question. Such measurements and evaluations may be made in qualitative and/or quantitative terms. Thus, for example, the characteristics or features of a disease, pathological condition or disorder and/or the symptoms of a disease, pathological condition or disorder may be reduced to any effect or in any amount. In the context of a subject suffering from an inflammatory disease, the terms 'treatment' and 'treat' refer to the medical management of a subject with the intention of curing, improving or stabilising said inflammatory disease.

As used herein, the term 'subject' includes, but is not limited to, animals, plants, and any other organism or entity. The subject may be a vertebrate, more specifically a mammal (e.g., a human being, a horse, a pig, a rabbit, a dog, a sheep, a goat, a non-human primate, a cow, a cat, a guinea pig, or a rodent), a fish, a bird, or a reptile or amphibian. The term does not denote a particular age or sex. Therefore, adult and newborn subjects, as well as foetuses, whether male or female, are covered by the definition of subject. A patient is a subject affected by a disease or disorder. The term 'patient' includes human and veterinary subjects. In another aspect of the invention, one or more compounds of formula **I** for use in the treatment of an inflammatory disease may be administered to a subject comprising a human or an animal, including, but not limited to, a mouse, a dog, a cat, a horse, a bovine or an ovine and the like.

As indicated above, compounds of formula **I** are capable of inhibiting the phosphorylating activity of Alk5 kinase, which inhibits the activation of macrophages, thereby inhibiting the inflammatory response.

The term 'inhibit' means to reduce or decrease the phosphorlylating activity of the Alk5 kinase. This may involve complete inhibition of the activity or expression of the kinase, or partial inhibition. Inhibition can be compared to a control or standard level. Inhibition can be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% compared to said control.

In a particular embodiment, the inflammatory disease is selected from the group consisting of diseases that progress with an inflammatory and/or pro-fibrotic component.

In another particular embodiment, the inflammatory disease is a pulmonary inflammatory disease selected from the group consisting of acute respiratory distress syndrome, chronic obstructive pulmonary disease, pulmonary fibrosis, pulmonary hypertension, pulmonary inflammation, preferably acute respiratory distress syndrome and pulmonary fibrosis.

In another particular instance, inflammatory disease is an inflammatory bowel disease that presents with a strong inflammatory component, such as Crohn's disease and ulcerative colitis.

In another particular embodiment, the inflammatory disease is a cardiovascular inflammatory disease that presents with a strong inflammatory component, such as acute myocardial infarction, myocarditis, or atherosclerosis.

In another particular embodiment, inflammatory disease is any inflammatory disease that has a strong inflammatory component, such as type 2 diabetes, multiple sclerosis, or cancer.

In another particular embodiment, the compound of formula **I** is ethyl 5-(imidazo[1,2-a]pyridin-3-yl)-1H-indole-2-carboxylate (31) (PS1801).

In another particular embodiment, the compound of formula **I** is ethyl 5-(imidazo[1,2-a]pyridin-3-yl)-1H-indole-2-carboxylate (31) (PS1906).

In a particular embodiment, the concentration of PS1801 or PS1906 administered to a subject is between 1000

mg/kg and 1 μg/kg of body weight, preferably between 500 mg/kg and 2 μg/kg, more preferably between 100 mg/kg and 3 μg/kg, even more preferably between 50 mg/kg and 4 μg/kg, even more preferably between 25 mg/kg and 5 μg/kg, even more preferably between 20 mg/kg and 8 μg/kg, even more preferably between 15 mg/kg and 10 μg/kg, even more preferably between 1 mg/kg and 50 μg/kg, and even more preferably between 800 μg/kg and 80 μg/kg.

Another additional object of the invention relates to a pharmaceutical composition comprising one or more compounds of formula **I** as defined above and one or more additional active compounds, and/or one or more pharmaceutically acceptable carriers, adjuvants or vehicles.

"Pharmaceutically acceptable" means a material that can be administered to a subject together with the selected compound without causing any undesirable biological effects or interacting deleteriously with any of the other components of the pharmaceutical composition in which it is contained.

In a further embodiment, the pharmaceutically acceptable vehicle may be a pharmaceutically acceptable disintegrant, surfactant, binder, and lubricant.

In a particular embodiment, the pharmaceutical composition comprises another additional active compound, such as steroids or corticosteroids (e.g., dexamethasone), and non-steroidal anti-inflammatory drugs (NSAIDs) such as salicylates, ibuprofen, naproxen.

In one particular embodiment, when the compound of formula **I** is used for Acute Respiratory Distress Syndrome, the other active ingredient is Dexamethasone.

In another particular embodiment, the additional active compound is another compound of formula **I,** as described above.

As used herein, the term "a combination of," when referring to two or more additional compounds, agents, or active pharmaceutical ingredients, means the administration of two or more compounds, agents, or active pharmaceutical ingredients to the subject before, simultaneously, or after each other.

In a particular embodiment, the additional active compound is selected from a mucolytic agent, a bronchodilator, an antibiotic, an antiviral agent, an anti-inflammatory agent, a nutritional agent, and combinations thereof.

The compounds of formula I of the invention and pharmaceutically acceptable compositions may be administered to a subject orally, intravenously, rectally, parenterally, intracisternally, intravaginally, intraperitoneally, topically (such as powders, ointments or drops), buccally, as an oral or nasal spray, or the like, depending on the severity of the infection being treated, preferably intravenously. In certain embodiments, the compounds or compositions of the invention may be administered at dosage levels of approximately 0.01 mg/kg to approximately 50 mg/kg and preferably approximately 1 mg/kg to approximately 25 mg/kg of the subject's body weight per day, once or more times per day, to obtain the desired therapeutic effect.

Another additional object of the invention relates to a pharmaceutical composition comprising one or more compounds of formula I as defined above and

one or more additional active compounds, and/or

one or more pharmaceutically acceptable carriers, adjuvants or vehicles

for use in the treatment of an inflammatory disease.

Each of the terms "comprises", "essentially consists of" and "consists of" may be substituted with any of the other two terms. The terms "a" or "an" may refer to one or a plurality of the elements they modify (e.g., "a reagent" may mean one or more reagents), unless it is clear from the context that one or more of the elements is being described. The term "approximately", as used herein, refers to a value within 10% of the underlying parameter (i.e., plus or minus 10%; for example, a weight of "approximately 100 grams" may include a weight between 90 grams and 110 grams). The use of the term "approximately" at the beginning of a list of values modifies each of the values (e.g., "approximately 1, 2, and 3" refers to "approximately 1, approximately 2, and approximately 3"). When describing a list of values, the list includes all intermediate values and all fractional values thereof (e.g., the list of values "80%, 85% or 90%" includes the intermediate value 86% and the fractional value 86.4%). When a list of values is followed by the term "or more", the term "or more" applies to each of the listed values (for example, the list "80%, 90%, 95% or more" or "80%, 90% or 95% or more" refers to "80% or more, 90% or more, or 95% or more"). When describing a list of values, the list includes all ranges between two of the listed values (for example, the list of "80%, 90% or 95%" includes ranges from "80% to 90%", "80% to 95%" and "90% to 95%"). Below are some examples of how the technology is applied.

Among the advantages of the present invention are:

advantages both in terms of production, as they are chemically synthesised compounds that have been optimised in the laboratory, maintaining identical solubility, stability and reproducible characteristics across product batches. This optimisation should result in both economic advantages, as synthesis is more economical than obtaining the compounds from natural products, and speed in the production of the compounds. The dose used in in vitro tests shows that very little

product is required to obtain the desired effect, and at this dose no toxic effect has been observed in the analysed cells.

**Brief description of the figures**

**Figure 1:** Graphical representation of the percentage inhibition of Alk5 kinase in vitro of different compounds synthesised in the present invention. (The commercial compound GW788388 (GW) was used as a control).

**Figure 2:** Evaluation of cell viability (cytotoxicity) on human THP-1-XBlue™-CD14 macrophages of the different compounds at three different concentrations (1, 5, and 10 μM), the vehicle is PBS. GW788388 (GW) was used as a control.***p<0.001

**Figure 3:** Effect of compounds PS1906 and PS1801 on the viability of human epithelial cells (Caco-2) and macrophages (THP-1-XBlue™-CD14) at different concentrations (1, 5, and 10 μM). GW788388 (GW) was used as a control.

**Figure 4:** Evaluation of the anti-inflammatory effect in THP-1-XBlue™-CD14 macrophages stimulated with bacterial LPS and subsequently treated with compounds PS1906 and PS1801 at 5 μM. GW788388 (GW) was used as a control. ****p<0.0001

**Figure 5:** Evaluation of anti-inflammatory effect in THP-1-XBlue™-CD14 macrophages without prior stimulation with bacterial LPS and treated with compounds PS1906 and PS1801 at 5 μM. GW788388 (GW) was used as a control.

**Figure 6:** In vivo evaluation (mouse model with acute lung injury (ALI) caused by exposure to bacterial LPS) of the protective effect of compound PS1906 at different equipotent doses (D1 and D2), where D1 = 156 μg/kg and D2 = 312 μg/kg. A) Histological images of mouse lungs stained with hematoxylin and eosin; B) Graphical representation of lung damage assessment (ALI Score) in the different treatments; C) Neutrophil count per mL of bronchoalveolar lavage fluid from the mice analyzed (represented on a logarithmic scale Log10); D) Lymphocyte count per mL of bronchoalveolar lavage fluid from the mice analyzed (represented on a logarithmic scale Log10). The data were represented as the mean value ± standard error of the mean. Where # p<0.01; ## p<0.001; ***p<0.0001 analyzed using one-way ANOVA adjusted with Dunnet's multiple comparison method.

**EXAMPLES**

**MATERIALS AND METHODS**

**A. OBTAINING AND CHARACTERIZATION OF CHEMICAL COMPOUNDS**

Synthesis of imidazo[1,2-a]pyridine derivatives

**Imidazo[1,2-a]pyridine-5-carboxylic acid (2)**

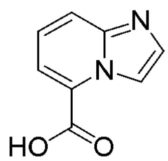

A reaction mixture of 2-chloroacetyl (13 mg, 0.173 mmol) and 6-aminopicolinic acid (20 mg, 0.144 mmol) in isopropanol (1.5 mL) is irradiated with microwaves in an inert atmosphere. After 40 min at 170 °C under microwave irradiation, the resulting solid is filtered and dried to obtain 25 mg, 43% yield.

**$^1$H NMR** (400 MHz, Methanol-$d_4$) δ 9,22 (d, *J* = 2,2 Hz, 1H); 8,29 (dd, *J* = 7,2, 1,3 Hz, 1H); 8,21 - 8,13 (m, 2H); 8,06 (dd, *J* = 9,0; 7,3 Hz, 1H).

**$^{13}$C NMR** (101 MHz, Methanol-$d_4$) δ 161,7; 140,9; 132,3; 129,5; 122,8; 121,5; 116,5; 115,9.

**Ethyl imidazo[1,2-a]pyridine-5-carboxylate (3)**

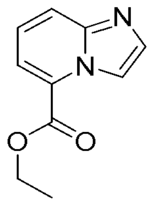

Thionyl chloride (0.072 mL, 0.987 mmol) is slowly added to a solution, in a sealed tube, of imidazo[1,2-a]pyridine-5-carboxylic acid (64 mg, 0.395 mmol) in EtOH (7 mL). After 24 hours at 78 °C, the solvent is removed under reduced pressure and the reaction mixture is neutralized using an aqueous solution of $NaHCO_3$. The crude reaction mixture is extracted using AcOEt as the organic solvent. The organic phases are dried over $MgSO_4$ and the solvent is removed under reduced pressure to obtain 50 mg, 66% yield.

**$^1$H NMR** (400 MHz, Acetone-$d_6$) δ 8,82 (s, 1H); 7,86 (dd, *J* = 10,7; 8,2 Hz, 2H); 7,75 (s, 1H); 7,36 (dd, *J* = 8,9; 7,2 Hz, 1H); 4,49 (q, *J* = 7,1 Hz, 2H); 1,44 (t, *J* = 7,1 Hz, 3H).

**$^{13}$C NMR**(101 MHz, Acetone-$d_6$) δ 162,7; 147,0; 135,6; 127,2; 123,50; 123,3; 119,5; 115,4; 62,8; 14,6.

**3-Phenylimidazo[1,2-a]pyridine (4)**

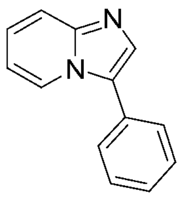

A reaction mixture of imidazo[1,2-a]pyridine (115 mg, 0.973 mmol), bromobenzene (0.204 mL, 1.946 mmol), Pd (AcO) (0.027 mg, 0.097 mmol), and KOAc (0.200 mg, 1.946 mmol) in DMF (4 mL) is irradiated with microwaves in an inert atmosphere. After 1 hour at 160 °C, the solvent is removed under reduced pressure, and the reaction mixture is extracted using AcOEt as the organic solvent. The organic phases were dried over $MgSO_4$ and the solvent was removed under reduced pressure. The crude reaction product was purified on a chromatographic column using a mixture of toluene/-MeOH (10:1) to obtain 147 mg, 77% yield.

**$^1$H NMR** (400 MHz, Chloroform-d) δ 8,34 (d, *J* = 7,0 Hz, 1H); 7,74 - 7,64 (m, 2H); 7,54 (dt, *J* = 15,4; 7,4 Hz, 4H); 7,46 - 7,39 (m, 1H); 7,25 - 7,15 (m, 1H); 6,81 (t, *J* = 6,8 Hz, 1H).

**$^{13}$C NMR** (101 MHz, Chloroform-d) δ 146,3; 132,7; 129,5; 129,4; 128,3; 128,2; 125,9; 124,4; 123,5; 118,4; 112,7.

**Imidazo[1,2-a]pyridin-5-ylmethanol (5)**

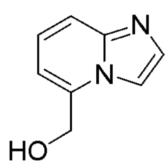

MeOH (13 μL, 0.325 mmol) is slowly added under an inert atmosphere to a mixture of ethyl imidazo[1,2-a]pyridine-5-carboxylate (30 mg, 0.158 mmol) and $LiBH_4$ (7 mg, 0.325 mmol) in $Et_2O$ (3 mL). After 5 hours at room temperature, the reaction mixture was saturated with a 2M HCI solution at 0 °C. The reaction mixture is extracted with AcOEt as the organic solvent, the organic phases are dried over $MgSO_4$, and the solvent is removed under reduced pressure to obtain the desired product.

**$^1$H NMR** (400 MHz, DMSO-d6) δ 7,88 (d, J = 1,3 Hz, 1H); 7,63 (d, J = 1,3 Hz, 1H); 7,53 (d, J = 9,0 Hz, 1H); 7,26 (dd,

J = 9,1; 6,8 Hz, 1H); 6,92 (d, J = 6,6 Hz, 1H); 4,76 (s, 2H).

**$^{13}$C NMR**(101 MHz, DMSO) δ 145,0; 138,4; 133,2; 124,2; 115,5; 110,5; 109,4; 59,6.

**2-(4-Methoxyphenyl)-6-methylimidazo[1,2-a]pyridine (8)**

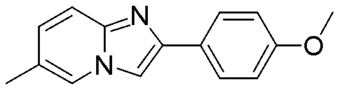

Under an inert atmosphere, 2-bromo-1-(4-methoxyphenyl)ethan-1-one (160 mg, 0.694 mmol) is added to a solution of 5-methylpyridin-2-amine (50 mg, 0.462 mmol) in acetone (4 mL). After 18 hours at 60 °C, the solvent is removed under reduced pressure and the reaction mixture is alkalinized with an aqueous solution of NaOH (1M). The reaction mixture is extracted using $CH_2Cl_2$ as the organic solvent, the organic phases are dried over $MgSO_4$, and the solvent is removed under reduced pressure. The crude reaction product is purified on a chromatographic column using a mixture of Hex/AcOEt (1:1) as the eluent to obtain 72 mg, 65% yield.

**$^1$H NMR** (400 MHz, DMSO-d6) δ 8,28 (s, 1H); 8,17 (s, 1H); 7,87 (d, *J* = 8,7 Hz, 2H); 7,45 (d, *J* = 9,2 Hz, 1H); 7,07 (dd, J = 9,2; 1,8 Hz, 1H); 6,99 (d, *J* = 8,7 Hz, 2H); 3,79 (s, 3H); 2,27 (s, 3H).

**$^{13}$C NMR**(101 MHz, DMSO-*d*6) δ 158,9; 144,2; 143,8; 127,5; 126,7; 126,7; 124,1; 121,15; 115,8; 114,1; 107,7; 55,1; 17,5.

Experimental procedure A: Cross-coupling reaction (A1) and heterocyclization (A2)

**A1**

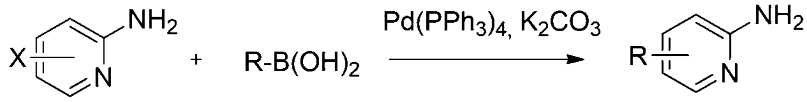


**2** **3** **4**

X = Cl, Br, I (positions 4 and 5)

R = Aryl (*E*)-styrenyl

R = Aryl (*E*)-styrenyl

**A2**

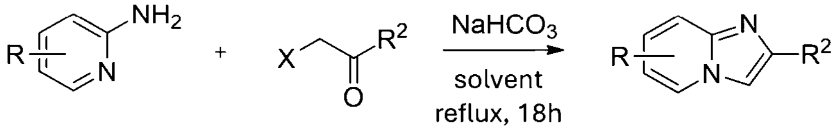


**4** **5** **1**

R = Aryl (*E*)-styrenyl

X = Cl, Br
$R^2$ = H, Aryl

Formula I
R = $R^6$, $R^7$ (Aryl, (*E*)-styrenyl)
$R^2$ = H, Aryl

Suzuki reaction (A1):

The corresponding 4- or 5-halo-2-aminopyridine (1 eq) is added to a mixture of the corresponding boronic acid (1.2 eq), $K_2CO_3$ (1.3 eq), and $Pd(PPh_3)_4$ (2% mol) in a mixture of DME/$H_2O$ (4:1, 5 mL/mmol) under an inert atmosphere in a microwave tube. After 30 minutes at 110 °C, the solvent is evaporated under reduced pressure and the crude reaction product is purified on a chromatographic column using the corresponding eluents.

Alternative to procedure through conventional heating

Alternatively, the corresponding 4- or 5-halo-2-aminopyridine (1 eq) is added to a mixture of the corresponding boronic acid (1.6 eq), $Na_2CO_3$ (2 eq), and $Pd(PPh_3)_4$ (2-10% mol) in a dioxane/$H_2O$ mixture (12:1, 20 mL/mmol), and the

mixture is kept under reflux under argon for 18 h. After evaporating the solvent under reduced pressure, the crude reaction mixture is purified on a chromatographic column using the corresponding eluents.

Heterocyclization (A2):

The corresponding α-halo ketone (1.75 eq) is added to a mixture of the 2-substituted aminopyridine (1 eq) and $NaHCO_3$ (0.1 g/5 mL) in EtOH (4 mL). After 18 hours at 90°C, the solvent was evaporated under reduced pressure ,the resulting solid was suspended in a solution of $K_2CO_3$ in $H_2O$ and extracted using AcOEt as the organic phase. The organic phases were dried over $MgSO_4$ and the organic solvent was evaporated under reduced pressure to give the corresponding imidazopyridines.

**4-(Imidazo[1,2-a]pyridin-7-yl)phenol (12)**

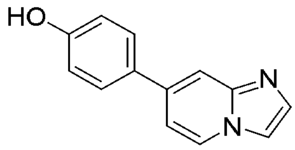

Using experimental method A2, a mixture of 4-(4-hydroxyphenyl)-2-aminopyridine (30 mg, 0.161 mmol), $NaHCO_3$ (83 mg, 0.1 g/5 mL EtOH), and 2-chloroacetaldehyde (20 μL, 0.322 mmol) in EtOH (4 mL) was stirred at 90 °C to give 25 mg, 74% yield.

**$^1$H NMR** (400 MHz, DMSO-$d_6$) δ 8,53 (d, *J* = 7,2 Hz, 1H); 7,89 (s, 1H), 7,70 (s, 1H); 7,61 (d, *J* = 8,2 Hz, 2H); 7,54 (s, 1H); 7,20 (d, *J* = 6,9 Hz, 1H); 6,86 (d, *J* = 8,3 Hz, 2H).

**$^{13}$C NMR(101** MHz, DMSO-$d_6$) δ 173,7; 158,6; 145,2; 136,2; 133,5; 127,5; 126,7; 116,0; 112,4; 111,3; 111,0.

**HPLC-MS (ES$^+$):** $CH_3CN/H_2O$ 2:98 a 95:5 (5 min), tr = 3,95 min, [M+H$^+$] = 211.

**4-(2-(4-(Trifluoromethyl)phenyl)imidazo[1,2-a]pyridin-7-yl)phenol (13)**

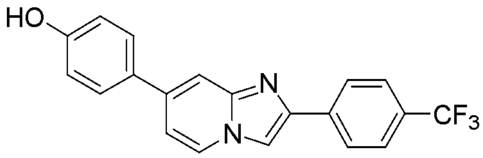

Using experimental method A2, a mixture of 4-(4-hydroxyphenyl)-2-aminopyridine (30 mg, 0.161 mmol), $NaHCO_3$ (83 mg, 0.1 g/5 mL EtOH), and 2-bromo-1-(4-(trifluoromethyl)phenyl) ethan-1-one (30 mg, 0.322 mmol) in EtOH (4 mL) was stirred at 90 °C. The crude reaction mixture was purified on a chromatographic column using $CH_2Cl_2$/MeOH as the eluent to obtain 31 mg, 54% yield.

**$^1$H NMR** (500 MHz, DMSO-$d_6$) δ 9,74 (s, 1H); 8,56 (d, *J* = 7,1 Hz, 1H); 8,52 (s, 1H); 8,19 (d, *J* = 8,1 Hz, 2H); 7,80 (d, *J* = 6,4 Hz, 3H); 7,68 (d, *J* = 8,6 Hz, 2H); 7,26 (dd, *J* = 7,2; 1,9 Hz, 1H); 6,89 (d, *J* = 8,6 Hz, 2H);

**$^{13}$C NMR(**126 MHz, DMSO-$d_6$) δ 158,4; 146,2; 143,6; 138,4; 137,5; 128,7; 128,2; 127,3; 126,4; 126,1 (q, $J_{CF}$ = 3,8 Hz); 124,8 (q, $J_{CF}$ = 271 Hz); 116,3; 112,2; 111,8; 110,5.

**HPLC-MS (ES$^+$):** $CH_3CN/H_2O$ 30: 70 a 95:5 (10 min), tr = 1,96 min, [M+H$^+$] = 355.

**4-(2-(4-(Trifluoromethyl)phenyl)imidazo[1,2-a]pyridin-6-yl)phenol (15)**

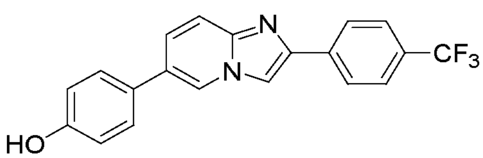

Using experimental method A2, a mixture of 5-(4-hydroxyphenyl)-2-aminopyridine (50 mg, 0.268 mmol), $NaHCO_3$ (83 mg, 0.1 g/5 mL EtOH), and 2-bromo-1-(4-(trifluoromethyl)phenyl) ethan-1-one (215 mg, 0.805 mmol) in EtOH (4 mL) was stirred at 90 °C. The crude reaction mixture was purified on a chromatographic column to obtain 31 mg, 31% yield.
**$^1$H NMR**(500 MHz, DMSO-*d*$_6$) δ 9,65 (s, 1H); 8,77 (dd, *J* = 1,9; 1,0 Hz, 1H); 8,52 (s, 1H); 8,20 (d, *J* = 7,8 Hz, 2H); 7,80 (d, *J* = 7,9 Hz, 2H); 7,65 (d, *J* = 9,4 Hz, 1H); 7,58 (dd, *J* = 9,4; 1,8 Hz, 1H); 7,56 - 7,53 (m, 2H); 6,89 (d, *J* = 8,6 Hz, 2H).
**$^{13}$C NMR** (126 MHz, DMSO-*d*$_6$) δ 157,4; 144,1; 142,9; 137,9; 127,7; 127,1; 126,0; 125,7; 125,6 (q, $J_{CF}$ = 3,8 Hz); 125,6; 124,3 (q, $J_{CF}$ = 272,5 Hz); 122,9; 116,7; 115,9; 110,8.
**HPLC-MS (ES$^+$):** $CH_3CN/H_2O$ 30:70 a 95:5 (10 min), tr = 2,91 min, [M+H$^+$] = 355.

**4-(2-(4-Methoxyphenyl)imidazo[1,2-a]pyridin-7-yl)phenol (16)**

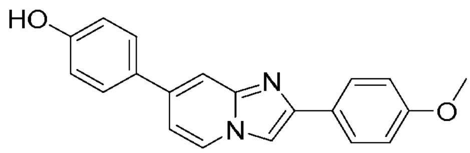

Using experimental method A2, a mixture of 4-(2-aminopyridin-4-yl)phenol (69 mg, 0.386 mmol) and 2-bromo-1-(4-(methoxy)phenyl) ethan-1-one (103 mg, 0.464 mmol) in acetone (5 mL) was stirred at 90 °C. The crude reaction mixture was filtered (0.22 μF) to give 82 mg, 67% yield.
**$^1$H NMR** (400 MHz, DMSO-*d*$_6$) δ 9,79 (s, 1H); 8,58 (d, *J* = 7,1 Hz, 1H); 8,33 (s, 1H); 7,89 (d, *J* = 8,7 Hz, 2H); 7,77 (s, 1H); 7,69 (d, *J* = 8,7 Hz, 2H); 7,34 (d, *J* = 7,1 Hz, 1H); 7,05 (d, *J* = 8,8 Hz, 2H); 6,91 (d, *J* = 8,6 Hz, 2H); 3,81 (s, 3H).
**$^{13}$C NMR** (101 MHz, DMSO-*d*$_6$) δ 159,4; 158,2; 144,2; 142,7; 127,9; 127,9; 127,0; 127,0; 124,7; 115,9; 114,3; 112,1; 109,6; 108,0; 55,2.
**HPLC-MS (ES$^+$):** $CH_3CN/H_2O$ 15:85 a 95:5 (5 min), tr = 4,09 min, [M+H$^+$] = 317.

**4-(2-(4-Methoxyphenyl)imidazo[1,2-a]pyridin-6-yl)phenol (17)**

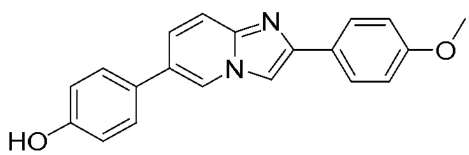

Using the experimental method A2, a mixture of 4-(6-aminopyridin-3-yl)phenol (70 mg, 0.375 mmol) and 2-bromo-1-(4-(methoxy)phenyl) ethan-1-one (103 mg, 0.464 mmol) in acetone (5 mL) was stirred at 90 °C. The crude reaction mixture was filtered to obtain 71 mg, 60% yield.
**$^1$H NMR** (400 MHz, DMSO-*d*$_6$) δ 9,64 (s, 1H); 8,76 (s, 1H); 8,30 (s, 1H); 7,90 (d, *J* = 8,7 Hz, 2H); 7,61 (m, 2H); 7,54 (d, *J* = 8,5 Hz, 2H); 7,04 (d, *J* = 8,8 Hz, 2H); 6,89 (d, *J* = 8,6 Hz, 2H); 3,81 (s, 3H).
**$^{13}$C NMR** (126 MHz, DMSO-*d*$_6$) δ 159,2; 157,4; 143,1; 127,7; 127,0; 126,9; 125,8; 125,7; 122,8; 115,8; 115,6; 114,2; 108,5; 55,1.
**HPLC-MS (ES$^+$):** $CH_3CN/H_2O$ 15:85 a 95:5 (5 min), tr = 4,13 min, [M+H$^+$] = 317.

**(E)-7-Styrylimidazo[1,2-a]pyridine (19)**

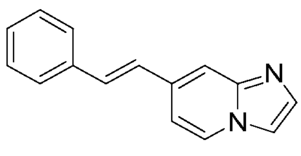

Using experimental method A2, a mixture of (E)-4-styrylpyridine-2-amine (50 mg, 0.254 mmol) and 2-chloroacetaldehyde (19 µL, 0.305 mmol) in acetone (6 mL) was stirred at 40 °C to obtain 53 mg, 94% yield.

**$^1$H NMR** (400 MHz, Chloroform-d) δ 8,00 (d, *J* = 7,1 Hz, 1H); 7,54 (d, *J* = 8,0 Hz, 2H); 7,46 (d, *J* = 8,8 Hz, 3H); 7,31 (t, *J* = 7,5 Hz, 2H); 7,26 - 7,17 (m, 2H); 7,03 (dd, *J* = 16,5; 6,5 Hz, 3H).

**$^{13}$C NMR** (101 MHz, Chloroform-d) δ 145,8; 136,7; 134,4; 134,1; 130,6; 128,9; 128,3; 126,8; 125,5; 115,7; 112,6; 110,2.

**HPLC-MS (ES$^+$):** $CH_3CN/H_2O$ 5:95 a 95:5 (5 min), tr = 4,59 min, [M+H$^+$] = 221.

**(E)-2-(4-Methoxyphenyl)-7-styrylimidazo[1,2-a]pyridine (20)**

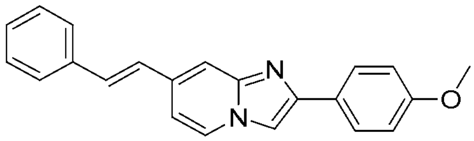

Using experimental method A2, a mixture of (E)-4-styrylpyridin-2-amine (63 mg, 0.323 mmol) and 2-bromo-1-(4-(trifluoromethyl)phenyl) ethan-1-one (88 mg, 0.387 mmol) in acetone (2 mL) was stirred at 40 °C. The crude reaction mixture was filtered (0.22 µF) to give 60 mg, 47% yield.

**$^1$H NMR** (500 MHz, Chloroform-d) δ 7,98 (d, *J* = 7,0 Hz, 1H); 7,73 (d, *J* = 8,7 Hz, 2H); 7,67 (s, 1H); 7,44 (s, 1H); 7,39 (d, *J* = 7,1 Hz, 2H); 7,22 (t, *J* = 7,6 Hz, 2H); 7,14 (t, *J* = 7,3 Hz, 1H); 7,01 (d, *J* = 16,3 Hz, 1H); 6,98 - 6,92 (m, 2H); 6,81 (d, *J* = 8,8 Hz, 2H); 3,69 (s, 3H).

**$^{13}$C NMR** (126 MHz, Chloroform-d) δ 159,4; 145,5; 145,3; 136,2; 134,3; 130,2; 128,5; 127,9; 127,0; 126,4; 126,45; 125,7; 125,2; 114,3; 113,9; 109,9; 107,5; 55,1.

**HPLC-MS (ES$^+$):** $CH_3CN/H_2O$ 15:85 a 95:5 (5 min), tr = 4,81 min, [M+H$^+$] = 327.

**7-(3,4-Dimethoxyphenyl)imidazo[1,2-a]pyridine (21)**

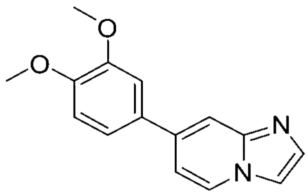

Using the A2 experimental method, a mixture of 4-(3,4-dimethoxyphenyl)pyridine-2-amine (50 mg, 0.217 mmol), $NaHCO_3$ (50 mg, 0.1 g/5 mL EtOH), and 2-chloroacetaldehyde (27 µL, 0.434 mmol) in EtOH (2.5 mL) was stirred at 90 °C to give 52 mg, 94% yield.

**$^1$H NMR** (400 MHz, Chloroform-d) δ 8,13 (d, *J* = 7,1 Hz, 1H); 7,76 (s, 1H); 7,63 (s, 1H); 7,55 (s, 1H); 7,20 (dd, *J* = 8,3; 2,2 Hz, 1H); 7,15 (d, *J* = 2,2 Hz, 1H); 7,04 (dd, *J* = 7,1; 1,8 Hz, 1H); 6,95 (d, *J* = 8,3 Hz, 1H); 3,95 (s, 3H); 3,92 (s, 3H).

**$^{13}$C NMR** (101 MHz, Chloroform-d) δ 149,5; 146,1; 137,4; 134,3; 131,6; 125,6; 119,3; 113,9; 112,3; 111,9; 111,7; 109,9; 56,1.

**HPLC-MS (ES$^+$):** $CH_3CN/H_2O$ 10:90 a 95:5 (5 min), tr = 1,46 min, [M+H$^+$] = 255.

**Experimental procedure B: Heterocyclization (B1) and Suzuki coupling reaction (B2)**

B1 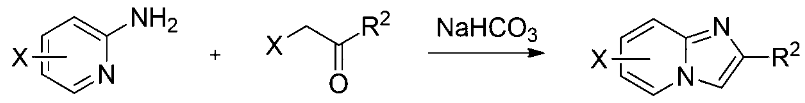


2 5 6

X = Cl, Br, I (in position 4 or 5)

X = Cl, Br
$R^2$ = H, Aryl

X = I, Br
$R^2$ = H, Aryl

B2 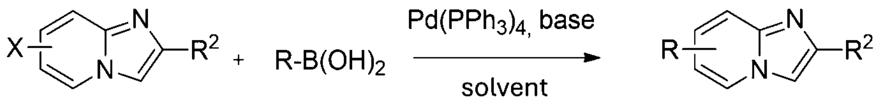


6 3 1

X = Cl, Br, I
$R^2$ = H, Aryl

R = (Aryl, (*E*)-styrenyl)

Formula I
R = $R^6$, $R^7$ (Aryl, (*E*)-styrenyl)
$R^2$ = H, Aryl

Heterocyclization (B1):

The corresponding α-halo-ketone (1.5-2 eq) is added to a mixture of the corresponding halo-2-aminopyridine and $NaHCO_3$ (0.1 g/5 mL-if EtOH was used as the solvent) in EtOH. The reaction mixture was heated to a temperature above the boiling point of the solvent used in each case for 18 hours. After the reaction time had elapsed, the solvent was evaporated under reduced pressure and the crude reaction mixture was suspended in an aqueous solution of $K_2CO_3$ and subsequently extracted using AcOEt. The organic phases were dried over anhydrous $MgSO_4$, filtered, and the solvent was evaporated under reduced pressure to obtain the corresponding imidazo[1,2-a]pyridines.

Suzuki reaction (B2):

The corresponding haloimidazo[1,2-a]pyridine (1 eq) is added to a mixture of the corresponding boronic acid (1.2 eq), and the corresponding base ($NaHCO_3$ (1.3 eq) or $K_2CO_3$ (1.1 eq)) and $Pd(PPh_3)_4$ (2 mmol%) in 1,4-dioxane/$H_2O$ (3:1) or DME/$H_2O$ (4:1) - depending on the reaction- in a microwave tube. The mixture is heated to 130 °C for 30 minutes. After the reaction time, the solvent was evaporated under reduced pressure and the crude reaction product was purified in a chromatographic column to obtain the corresponding imidazo[1,2-a]pyridines.

**6-Iodo-2-(4-(trifluoromethyl)phenyl)imidazo[1,2-a]pyridine (25)**

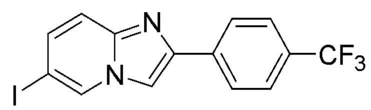

Using experimental method B1, a mixture of 5-iodo-2-aminopyridine (41 mg, 0.136 mmol), $NaHCO_3$ (80 mg, 0.1 g/5 mL EtOH), and 2-bromo-1-(4-(trifluoromethyl)phenyl) ethan-1-one (60 mg, 0.204 mmol) in EtOH (4 mL) was stirred at 90 °C and the crude reaction mixture was purified on a chromatographic column using $CH_2Cl_2$/MeOH as the eluent to obtain 25 mg, 47% yield.

**$^1$H NMR** (400 MHz, Chloroform-d) δ 8,40 (s, 1H); 8,03 (d, *J* = 8,0 Hz, 2H); 7,85 (s, 1H); 7,68 (d, *J* = 8,1 Hz, 2H); 7,45 (d, *J* = 9,4 Hz, 1H); 7,37 (dd, *J* = 9,5; 1,6 Hz, 1H).

**$^{13}$C NMR** (101 MHz, Chloroform-d) δ 144,6; 144,4; 136,6; 133,4; 126,3; 125,9 (q, $J_{CF}$ = 273 Hz); 124,3 (q, $J_{CF}$ = 3,8 Hz); 118,7; 108,7; 75,8.

**HPLC-MS (ES$^+$):** $CH_3CN/H_2O$ 40:60 a 95:5 (10 min), tr = 5,57, [M+H$^+$] = 389.

**7-(Benzofuran-2-yl)imidazo[1,2-a]pyridine. (26) PS1906**

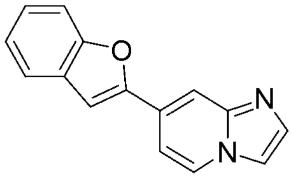

Using experimental method A1, 4-iodo-2-aminopyridine (440 mg, 2.15 mmol), benzofuran-2-boronic acid (584 mg, 3.6 mmol), $Pd(PPh_3)_4$, (248 mg, 10 mol%), and $Na_2CO_3$ (477 mg, 4.5 mmol) are successively added to a mixture of dioxane (74 mL) and $H_2O$ (6 mL). The mixture was kept under reflux under argon for 18 h. After removal of the solvent under vacuum, the crude reaction mixture was purified by column chromatography using $CH_2Cl_2$/MeOH as the eluent to obtain 431 mg, 95% yield. Using experimental method A2, the product obtained (5-(benzofuran-2-yl)pyridin-2-amine) was dissolved in EtOH (15 mL) and a solution of chloroacetaldehyde (40% in water, 1.0 mL) and $NaHCO_3$ (344 mg, 4.1 mmol) were added successively. The mixture was kept under reflux for 18 h. After dilution with ethyl acetate, filtration through celite, washing with saturated sodium chloride solution, drying of the organic phase over $MgSO_4$, filtration, and evaporation of the solvent under vacuum, the crude reaction mixture was purified on a chromatographic column using $CH_2Cl_2$/MeOH as the eluent to obtain 440 mg of compound 26. 92% yield.
Using experimental method B2, benzofuran-2-boronic acid (62 mg, 0.106 mmol), $K_2CO_3$ (46 mg, 0.106 mmol), $Pd(PPh_3)_4$, (6 mg, 2 mol%) and 7-bromoimidazo[1,2-a]pyridine (50 mg, 0.082 mmol) were dissolved in 5 mL of a mixture of DME/H2O (4:1). The crude reaction product was purified on a chromatographic column using $CH_2Cl_2$/MeOH as the eluent to obtain 42 mg, 70% yield.
**$^1$H NMR** (400 MHz, Chloroform-d) $\delta$ 8,09 - 8,00 (m, 2H); 7,62 (s, 1H); 7,55 - 7,43 (m, 3H); 7,29 - 7,21 (m, 1H); 7,20 - 7,11 (m, 2H); 7,01 (s, 1H).
**$^{13}$C NMR** (101 MHz, Chloroform-d) $\delta$ 155,2; 153,8; 145,5; 135,0; 129,0; 126,7; 125,8; 125,1; 123,3; 121,2; 113,0; 112,9; 111,4; 109,7; 103,2.
**HPLC-MS (ES$^+$):** $CH_3CN/H_2O$ 15:85 a 95:5 (5 min), tr = 1,39 min, [M+H$^+$] = 235.
The compound PS1906 also has an anti-inflammatory effect in an in vivo murine model of acute lung injury (ALI), where it shows large macroscopic differences and is significantly different from the group of sick mice treated with LPS. Flow cytometry analysis of bronchoalveolar lavage fluid showed a dose-dependent anti-inflammatory effect of the PS1906 compound on neutrophils, lymphocytes, and the total number of infiltrated cells.

**4-(Imidazo[1,2-a]pyridin-6-yl)phenol (27)**

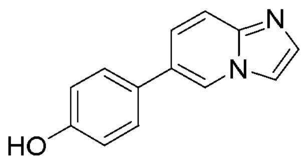

Using experimental method B2, 4-hydroxyphenyl boronic acid (20 mg, 0.147 mmol), $K_2CO_3$ (22 mg, 0.160 mmol), $Pd(PPh_3)_4$, (2 mg, 2 mmol%) and 6-iodoimidazo[1,2-a]pyridine (30 mg, 0.123 mmol) were dissolved in 3 mL of a DME/$H_2O$ mixture (4:1). The crude reaction product was purified on a chromatographic column using $CH_2Cl_2$/MeOH as the eluent to obtain 28 mg, 70% yield.
**$^1$H NMR** (400 MHz, DMSO-*d*$_6$) $\delta$ 9,60 (s, 1H); 8,78 (s, 1H); 7,93 (s, 1H); 7,63 - 7,55 (m, 2H); 7,55 - 7,48 (m, 3H); 6,87 (d, *J* = 8,6 Hz, 2H).
**$^{13}$C NMR** (101 MHz, DMSO) $\delta$ 157,2; 143,5; 133,3; 127,6; 127,3; 125,1; 124,4; 122,9; 116,7; 115,8; 113,3.
**HPLC-MS (ES$^+$):** $CH_3CN/H_2O$ 2:98 a 95:5 (5 min), tr = 3,92 min, [M+H$^+$] = 211.

**(E)-6-Styrylimidazo[1,2-a]pyridine (28)**

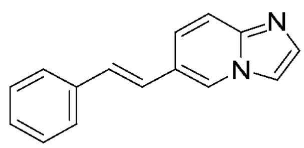

Using experimental method B2, styryl boronic acid (22 mg, 0.147 mmol), $NaHCO_3$ (11 mg, 0.135 mmol), $Pd(PPh_3)_4$, (2 mg, 2 mmol%) and 6-iodoimidazo [1,2-a]pyridine (30 mg, 0.123 mmol) were dissolved in 3 mL of a 1,4-dioxane/$H_2O$ (3:1) mixture. The crude reaction product was purified on a chromatographic column using $CH_2Cl_2$/MeOH as the eluent to obtain 25 mg, 91% yield.

**$^1$H NMR** (400 MHz, Chloroform-d) δ 8,05 (s, 1H); 7,57 - 7,50 (m, 2H); 7,47 (s, 1H); 7,46 - 7,36 (m, 3H); 7,34 - 7,25 (m, 2H); 7,25 - 7,17 (m, 1H); 6,99 (d, *J* = 16,3 Hz, 1H); 6,92 (d, *J* = 16,3 Hz, 1H).

**$^{13}$C NMR** (101 MHz, Chloroform-d) δ 145,1; 136,9; 134,1; 129,5; 128,9; 128,1; 126,5; 124,3; 124,2; 123,5; 122,4; 117,9; 112,9.

**HPLC-MS (ES$^+$):** $CH_3CN/H_2O$ 10:90 a 95:5 (5 min), tr = 4,13 min, [M+H$^+$] = 221.

**6-(Benzofuran-2-yl)imidazo[1,2-a]pyridine (29)**

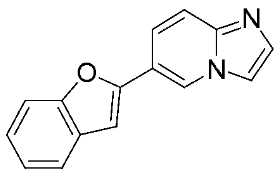

Using experimental method B2, benzofuran-2-boronic acid (16 mg, 0.106 mmol), $K_2CO_3$ (15 mg, 0.106 mmol), $Pd(PPh_3)_4$, (2 mg, 2 mmol%) and 6-iodoimidazo[1,2-a]pyridine (20 mg, 0.082 mmol) were dissolved in 3 mL of a DME/$H_2O$ mixture (4:1). The crude reaction product was purified by semi-preparative HPLC (gradient: 10:90 to 40:60 $CH_3CN/H_2O$ for 30 min) to obtain 10 mg, 52% yield.

**$^1$H NMR** (400 MHz, Acetone-*d*$_6$) δ 9,07 (s, 1H); 8,02 (s, 1H); 7,74 (dd, *J* = 9,5; 1,8 Hz, 1H); 7,70 - 7,60 (m, 3H); 7,56 (d, *J* = 8,1 Hz, 1H); 7,41 - 7,32 (m, 2H); 7,27 (t, *J* = 7,4 Hz, 1H).

**$^{13}$C NMR** (101 MHz, Acetone-*d*$_6$) δ 155,6; 153,9; 145,4; 135,0; 130,0; 125,6; 124,1; 123,9; 122,5; 121,9; 118,5; 117,0; 114,8; 111,7; 103,2.

**HPLC-MS (ES$^+$)**: $CH_3CN/H_2O$ 10:90 a 95:5 (5 min), tr = 4,40 min, [M+H$^+$] = 235.

**6-(3,4-Dimethoxyphenyl)imidazo[1,2-a]pyridine (30)**

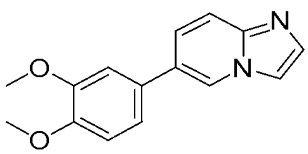

Using experimental method B2, 3,3,4-dimethoxyphenyl boronic acid (33 mg, 0.184 mmol), $Na_2CO_3$ (20 mg, 0.246 mmol), $Pd(PPh_3)_4$ (7 mg, 5 mmol%), and 6-iodoimidazo [1,2-a]pyridine (30 mg, 0.123 mmol) were dissolved in 3 mL of a mixture of $H_2O$/MeOH/Toluene (1:2:8). The crude reaction mixture was purified on a chromatographic column using $CH_2Cl_2$/MeOH as the eluent to obtain 30 mg, 96% yield.

**$^1$H NMR** (400 MHz, Chloroform-d) δ 8,25 (s, 1H); 7,64 (t, *J* = 10,9 Hz, 3H); 7,39 (dd, *J* = 9,3; 1,9 Hz, 1H); 7,09 (dd, *J* = 8,2; 2,2 Hz, 1H); 7,03 (d, *J* = 2,1 Hz, 1H); 6,95 (d, *J* = 8,2 Hz, 1H); 3,95 (s, 3H); 3,92 (s, 3H).

**$^{13}$C NMR** (101 MHz, Chloroform-d) δ 149,5; 149,2; 144,7; 134,0; 130,3; 126,9; 125,4; 122,6; 119,5; 117,7; 112,8; 111,8; 110,3; 56,1; 56,1.

**HPLC-MS (ES$^+$):** $CH_3CN/H_2O$ 10:90 a 95:5 (5 min), tr = 1,39 min, [M+H$^+$] = 255.

**Experimental procedure C: Heck reaction**

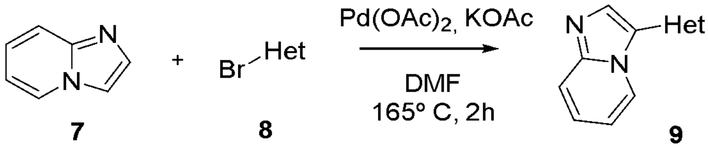


7 8 9

$Pd(OAc)_2$ (10 mmol%) is added to a solution of the corresponding imidazo[1,2-a]pyridine (1 eq), the corresponding haloheteroaryl (1.3 eq), and KOAc (2 eq) in DMF (4 mL) in a microwave tube under an argon atmosphere. After irradiating the tube at 165 °C for 2 hours, a concentrated KOH solution (1 mL) is added to the reaction mixture and left under stirring for 1 hour at room temperature. After the reaction time has elapsed, $H_2O$ is added and the reaction mixture is extracted with AcOEt. The organic phases are dried over $MgSO_4$ and the solvent is evaporated under reduced pressure. The crude reaction product is purified on a chromatographic column using a mixture of hexane/AcOEt to obtain the corresponding imidazo[1,2-a]pyridines.

**Ethyl 5-(imidazo[1,2-a]pyridin-3-yl)-1H-indole-2-carboxylate (31)**

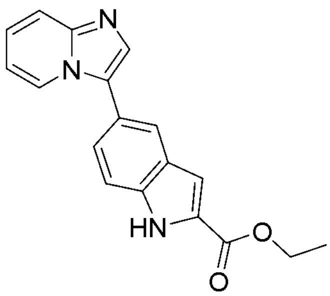

Using experimental method C, $Pd(OAc)_2$ (15 mg, 10 mmol%) is added to a solution of imidazo[1,2-a]pyridine (80 mg, 0.677 mmol), ethyl 5-bromo-1H-indole-2-carboxylate (0.235 mg, 0.880 mmol), and KOAc (86 mg, 0.880 mmol) in DMF (4 mL). The crude reaction mixture was purified on a chromatographic column using a mixture of hexane/AcOEt as the eluent to obtain 130 mg, 62% yield.

**$^1$H NMR** (400 MHz, Chloroform-d): δ 9,61 (s, 1H); 8,33 (d, *J* = 6,9 Hz, 1H); 7,86 (s, 1H); 7,70 (d, *J* = 11,5 Hz, 2H); 7,58 (d, *J* = 8,4 Hz, 1H); 7,48 (d, *J* = 8,5 Hz, 1H); 7,29 (s, 1H); 7,20 (t, *J* = 7,9 Hz, 1H); 6,80 (t, *J* = 6,9 Hz, 1H); 4,44 (q, *J* = 7,1 Hz, 2H); 1,43 (t, *J* = 7,1 Hz, 3H).

**$^{13}$C NMR** (101 MHz, Chloroform-d): δ 161,9; 145,8; 136,7; 132,1; 128,8; 128,1; 126,4; 126,0; 124,2; 123,5; 122,6; 121,8; 118,2; 113,0; 112,6; 108,7; 61,3; 14,5.

**HPLC-MS ($ES^+$):** $CH_3CN/H_2O$ 10:90 a 90:10 (5 min), tr = 4,24, [$M+H^+$] = 306.

**7-(Benzofuran-3-yl)imidazo[1,2-a]pyridine (32)**

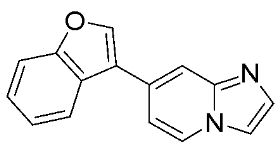

The synthesis of 32 was carried out using the general procedure B2. Using 7-bromoimidazo[1,2-a]pyridine (0.25 mmol), benzofuran-3-boronic acid (0.33 mmol) in $DME/H_2O$ (4:1). A yellowish-white solid was obtained with a yield of 82%.

**$^1$H NMR:** (500 MHz, $CDCl_3$) δ 8.21 (d, *J* = 7.0 Hz, 1H, 5), 7.98 (d, *J* = 0.9 Hz, 1H), 7.97 - 7.90 (m, 2H), 7.69 (s, 1H), 7.62 (s, 1H), 7.61 - 7.55 (m, 1H), 7.44 - 7.33 (m, 2H), 7.09 (d, *J* = 7.0 Hz, 1H).

**$^{13}$C NMR:** (126 MHz, $CDCl_3$) δ 156.32, 142.59, 134.44, 132.54, 128.94, 128.85, 126.31, 126.03, 125.45, 123.87,

120.73, 115.14, 112.96, 112.79, 112.41. MS ($ES^+$,m/z), 235 $[M+H]^+$.

**6-(Benzofuran-3-yl)imidazo[1,2-a]pyridine (33)**

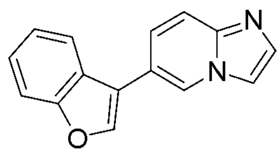

The synthesis of 33 was carried out using the general procedure B2. Using 6-iodoimidazo[1,2-a]pyridine (0.20 mmol), benzofuran-3-boronic acid (0.26 mmol) in DME/$H_2O$ (4:1). A dark green solid was obtained with a yield of 68%.

**$^1$H NMR:** (500 MHz, $CDCl_3$) δ 8.42 (s, 1H), 7.83 (s, 1H), 7.79 - 7.76 (m, 1H), 7.74 (d, *J* = 9.3 Hz, 1H), 7.69 (s, 1H), 7.67 (s, 1H), 7.58 (d, *J* = 8.2 Hz, 1H), 7.44 (dd, *J* = 9.3, 1.7 Hz, 1H), 7.42 - 7.38 (m, 1H), 7.35 (td, *J* = 7.6, 1.0 Hz, 1H).

**$^{13}$C NMR:** (126 MHz, $CDCl_3$) δ 155.9, 144.8, 141.7, 134.0, 126.1, 125.5, 125.2, 123.5, 123.2, 120.0, 118.6, 118.3, 117.9, 113.0, 112.2. MS ($ES^+$,m/z), 235 $[M+H]^+$.

**7-(Furan-3-yl)imidazo[1,2-a]pyridine (34)**

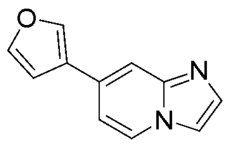

The synthesis of 34 was carried out using the general procedure B2. Using 7-bromoimidazo[1,2-a]pyridine (0.25 mmol), furan-3-boronic acid (0.33 mmol) in 1,4-dioxane/$H_2O$ (3:1). A brown oil was obtained with a yield of 73%.

**$^1$H NMR:** (500 MHz, $CDCl_3$) δ 8.26 (dd, *J* = 1.7, 1.0 Hz, 1H), 7.75 - 7.74 (m, 1H, 3), 7.71 (d, *J* = 9.4 Hz, 1H, 5), 7.66 (d, *J* = 1.3 Hz, 1H, 2), 7.61 (m, 1H), 7.53 - 7.52 (m, 1H), 7.37 (dd, *J* = 9.3, 1.7 Hz, 1H), 6.67 (dd, *J* = 1.9, 0.9 Hz, 1H).

**$^{13}$C NMR:** (126 MHz, $CDCl_3$) δ 144.29, 138.84, 132.13, 132.05, 128.5, 124.90, 122.14, 121.6 117.64, 112.76, 108.33. HPLC-MS ($ES^+$): $CH_3CN/H_2O$ 20:95 to 95:20, $t_r$ = 0.98 (92%); MS ($ES^+$,m/z), 185 $[M+H]^+$.

**6-(Furan-3-yl)imidazo[1,2-a]pyridine (35)**

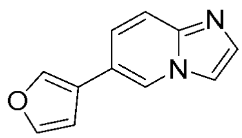

The synthesis of 35 was carried out using the general procedure B2. Using 6-iodoimidazo[1,2-a]pyridine (0.25 mmol), furan-3-boronic acid (0.32 mmol) in 1,4-dioxane/$H_2O$ (3:1). A brown oil was obtained with a yield of 58%.

**$^1$H NMR:** (500 MHz, $CDCl_3$) δ 8.11 (dd, *J* = 7.0, 1.0 Hz, 1H), 7.81 **(s,** 1H, **5),** 7.71 (m, 1H), 7.63 (d, *J* = 1.3 Hz, 1H, **2),** 7.56 (m, 1H), 7.52 (t, *J* = 1.7 Hz, 1H, **7),** 6.94 (dd, *J* = 7.0, 1.7 Hz, 1H), 6.78 - 6.77 (m, 1H).

**$^{13}$C NMR:** (126 MHz, $CDCl_3$) δ 146.1, 144.7, 139.8, 134.2, 126, 125.7, 125.2, 113.3, 112.7, 111.9, 108.8. HPLC-MS ($ES^+$): $CH_3CN/H_2O$ 2:50 to 50:2, $t_r$ = 1.67 (98%); MS ($ES^+$,m/z), 185 $[M+H]^+$.

**7-(Thiophen-3-yl)imidazo[1,2-a]pyridine (36)**

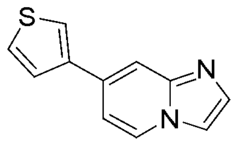

The synthesis of 36 was carried out using the general procedure B2. Using 7-bromoimidazo[1,2-a]pyridine (0.30 mmol), thiophene-3-boronic acid (0.39 mmol) in 1,4-dioxane/$H_2O$ (3:1). A dark red solid was obtained with a yield of 87%.
**$^1$H NMR:** (500 MHz, $CDCl_3$) δ 8.12 (d, *J* = 7.1 Hz, 1H), 7.82 (s, 1H), 7.65 (s, 1H), 7.58 - 7.54 (m, 2H), 7.45 - 7.42 (m, 2H), 7.07 (d, *J* = 7.1 Hz, 1H).
**$^{13}$C NMR:** (126 MHz, $CDCl_3$) δ 146.0, 140.1, 134.4, 132.3, 127.1, 125.9, 125.8, 121.6, 113.7, 112.3, 112.1. HPLC-MS ($ES^+$): $CH_3CN/H_2O$ 2:50 to 50:2, $t_r$ = 4.41 (99%); MS ($ES^+$,m/z), 200 $[M+H]^+$.

**6-(Thiophen-3-yl)imidazo[1,2-a]pyridine (37)**

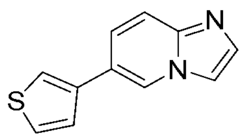

The synthesis of 37 was carried out using the general procedure B2. Using 6-iodoimidazo[1,2-a]pyridine (0.25 mmol), thiophene-3-boronic acid (0.32 mmol) in 1,4-dioxane/$H_2O$ (3:1). A grayish-brown solid was obtained with a yield of 82%.
**$^1$H NMR:** (500 MHz, $CDCl_3$) δ 8.16 (s, 1H), 7.47 - 7.43 (m, 4H), 7.30 - 7.23 (m, 1H), 7.15 (s, 1H), 7.08 (s, 1H).
**$^{13}$C NMR:** (126 MHz, $CDCl_3$) δ 144.7, 138.2, 134.1, 132.2, 127.2, 125.8, 125.0, 122.5, 120.9, 117.9, 112.9. HPLC-MS ($ES^+$): $CH_3CN/H_2O$ 5:95 to 95:5, $t_r$ = 4.21 (99%); MS ($ES^+$,m/z), 200 $[M+H]^+$.

**7-(Pyridin-4-yl)imidazo[1,2-a]pyridine (38)**

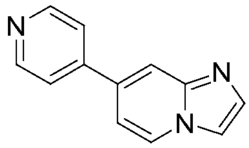

The synthesis of 38 was carried out using the general procedure B2. Using 7-bromoimidazo[1,2-a]pyridine (0.41 mmol), pyridinyl-4-boronic acid (0.52 mmol) in 1,4-dioxane/$H_2O$ (3:1). A light brown solid was obtained with a yield of 61%.
**$^1$H NMR:** (500 MHz, $MeOD_4$) δ 8.65 (d, *J* = 6.4 Hz, 2H), 8.59 (d, *J* = 7.1 Hz, 1H, **5),** 7.99 (s, 1H), 7.95 (s, 1H, 3), 7.86 - 7.83 (m, 2H), 7.69 (s, 1H), 7.37 (dd, *J* = 7.2, 1.8 Hz, 1H).
**$^{13}$C NMR:** (126 MHz, $MeOD_4$) δ 150.9, 148.0, 146.4, 136.1, 134.7, 128.6, 122.8, 115.3, 114.9, 112.6. HPLC-MS (ES+): $CH_3CN/H_2O$ 2:95 to 95:2, $t_r$ = 0.96 (97%); MS ($ES^+$,m/z), 196 $[M+H]^+$.

**6-(Pyridin-4-yl)imidazo[1,2-a]pyridine (39)**

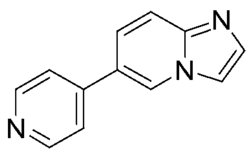

The synthesis of 20 was carried out using the general procedure B2. Using 6-iodoimidazo[1,2-a]pyridine (0.33 mmol), pyridinyl-4-boronic acid (0.42 mmol) in 1,4-dioxane/$H_2O$ (3:1). A purple solid was obtained with a yield of 70%.

**$^1$H NMR:** (500 MHz, $MeOD_4$) δ 9.03 - 9.01 (m, 1H), 8.63 (dd, $J$ = 4.7, 1.6 Hz, 2H), 7.98 (s, 1H), 7.79 (dd, $J$ = 4.7, 2.0 Hz, 2H), 7.74 (dd, $J$ = 9.4, 1.8 Hz, 1H, 7), 7.69 (d, $J$ = 9.4 Hz, 1H), 7.65 (d, $J$ = 1.4 Hz, 1H).

**$^{13}$C NMR:** (126 MHz, $MeOD_4$) δ 150.8, 146.9, 146.0, 134.2, 126.9, 125.9, 124.9, 122.8, 117.9, 115.4. HPLC-MS ($ES^+$): $CH_3CN/H_2O$ 2:95 to 95:2, $t_r$ = 0.71 (95%); MS ($ES^+$,m/z), 196 $[M+H]^+$.

**7-(Dibenzothiophene-3-yl)imidazo[1,2-a]pyridine (40)**

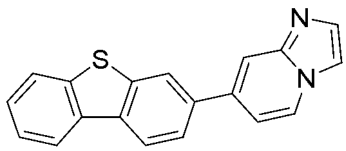

The synthesis of 40 was carried out using the general procedure B2. Using 7-bromoimidazo[1,2-a]pyridine (0.41 mmol), dibenzothiophene-4-boronic acid (0.52 mmol) in 1,4-dioxane/$H_2O$ (3:1). An orange solid was obtained with a yield of 90%.

**$^1$H NMR:** (500 MHz, $CDCl_3$) δ 8.25 (dd, $J$ = 7.0, 0.5 Hz, 1H), 8.21 - 8.19 (m, 2H), 8.02 (d, $J$ = 0.6 Hz, 1H), 7.86 - 7.84 (m, 1H), 7.73 (d, $J$ = 1.2 Hz, 1H), 7.67 - 7.66 (m, 1H), 7.60 - 7.60 - 7.57 (m, 1H), 7.55 (dd, $J$ = 7.4, 1.4 Hz, 1H), 7.49 - 7.47 (m, 2H), 7.22 (dd, $J$ = 7.0, 1.8 Hz, 1H).

**$^{13}$C NMR:** (126 MHz, $CDCl_3$) δ 145.91, 139.73, 138.66, 137.30, 136.96, 135.95, 134.92, 132.52, 128.93, 127.44, 127.11, 126.12, 125.63, 124.98, 123.08, 121.63, 117.01, 113.59, 112.80. HPLC-MS ($ES^+$): $CH_3CN/H_2O$ 15:95 to 95:15, $t_r$ = 4.50 (96%); MS ($ES^+$,m/z), 301 $[M+H]^+$.

**6-(Dibenzothiophene-3-yl)imidazo[1,2-a]pyridine (41)**

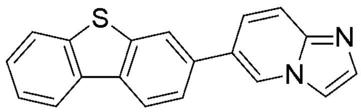

The synthesis of 41 was carried out using the general procedure B2. Using 6-iodoimidazo[1,2-a]pyridine (0.33 mmol), dibenzothiophene-4-boronic acid (0.42 mmol) in 1,4-dioxane/$H_2O$ (3:1). A light yellow solid was obtained with a yield of 69%.

**$^1$H NMR:** (500 MHz, $CDCl_3$) δ 8.50 (p, $J$ = 1.3 Hz, 1H), 8.23 - 8.19 (m, 2H), 7.87 - 7.84 (m, 1H), 7.78 (d, $J$ = 9.4 Hz, 1H), 7.73 (s, 1H), 7.68 (s, 1H), 7.61 - 7.53 (m, 2H), 7.51 - 7.48 (m, 3H).

**$^{13}$C NMR:** (126 MHz, $CDCl_3$) δ 144.80, 139.20, 138.80, 136.51, 135.65, 134.25, 132.71, 132.13, 127.14, 126.94, 126.08, 125.77, 125.27, 124.69, 124.54, 122.71, 121.88, 121.17, 117.93, 112.94. HPLC-MS ($ES^+$): $CH_3CN/H_2O$ 30:95 to 95:30, $t_r$ = 1.17 (94%); MS ($ES^+$,m/z), 301 $[M+H]^+$.

**Synthesis of ethyl 5-(7-(benzofuran-2-yl)-3-imidazo[1,2-a]pyridin)-1H-indole-2-carboxylate, (42)**

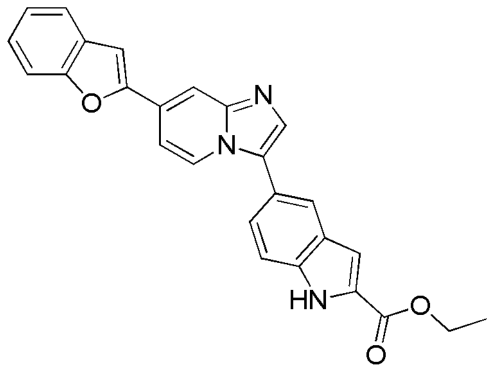

7-(benzofuran-2-yl)imidazo[1,2-a]pyridine (0.21 mmol) and ethyl 5-bromo-1H-indole-2-carboxylate (0.28 mmol) are dissolved in DMF (6 mL) in a microwave tube under a nitrogen flow. Pd $(OAc)_2$ (10 mmol%) is added. After 2 hours under microwave irradiation at 165 °C, a concentrated KOH solution (1 mL) is added to the reaction mixture. After 1 hour at room temperature, $H_2O$ is added and the reaction mixture is extracted with AcOEt. The organic phases were dried over anhydrous $MgSO_4$, filtered, and the solvent was evaporated under reduced pressure. The crude reaction product was purified on a chromatographic column using a mixture of hexane/AcOEt (1:4) as the eluent to obtain the corresponding product (42) as a yellow solid with a yield of 33%.

**$^1$H NMR:** (500 MHz, $CDCl_3$) δ 9.38 (s, 1H) 8.36 (d, J = 7.2 Hz, 1H), 8.18 (s, 1H), 7.89 (s, 1H), 7.77 (s, 1H), 7.62 - 7.59 (m, 2H), 7.56 (d, J = 8.2 Hz, 1H), 7.51 (dd, J = 8.5, 1.7 Hz, 1H), 7.35 - 7.30 (m, 2H), 7.27 - 7.23 (m, 2H), 7.11 (s, 1H), 4.45 (q, J = 7.1 Hz, 2H), 1.45 (t, J = 7.1 Hz, 3H).

**$^{13}$C NMR:** (126 MHz, $CDCl_3$) δ 162.0, 155.2, 154.0, 145.9, 136.7, 133.7, 129.1, 128.9, 128.0, 127.1, 126.3, 126.0, 125.2, 123.5, 123.4, 122.5, 121.6, 121.3, 113.2, 111.5, 109.8, 109.6, 108.8, 103.2, 61.2, 14.3. HPLC-MS ($ES^+$): $CH_3CN/H_2O$ 40:95 to 95:40, $t_r$ = 1.10 (91%); MS ($ES^+$,m/z), 422 $[M+H]^+$.

**B. EVALUATION OF TGFβ-1R (ALK5) KINASE INHIBITORY ACTIVITY**

The following chemically synthesized compounds (Table 1) were analyzed to determine their inhibitory activity in vitro by measuring the phosphorylation capacity of the Alk5 kinase (TGFβRI), following the instructions of Roth et al. (2010) (Roth GJ, Heckel A, Brandl T, Grauert M, Hoerer S, Kley JT, et al. Design, Synthesis, and Evaluation of Indolinones as Inhibitors of the Transforming Growth Factor β Receptor I (TGFβRI). J Med Chem.2010;53(20):7287-95; https://doi.org/10.1021/jm100812a).

Table 1. Initial list of compounds analyzed

| Molecular Formula / MW | Reference |
|---|---|
| $C_{18}H_{15}N_3O_2$ 305.337 | 31/ PS1801 |
| $C_{20}H_{16}N_2O_2$ 316.36 | 16 |
| $C_{13}H_{10}N_2O$ 210.236 | 12 |
| $C_{15}H_{10}N_{20}$ 234.258 | 26/ PS1906 |

For this purpose, commercially available recombinantAlk5 protein (Abcam, ref. ab105908) was used, as well as the commercial Kinase-Glo® Luminiscent kinase assay platform (Promega, ref. TB372), which determines the adenosine triphosphate (ATP) not consumed in the phosphorylation reaction by its conjugation to luciferin in a reaction catalyzed by the luciferase enzyme in the presence of $Mg^{2+}$. The luminescence observed is inversely proportional to kinase activity. The compound GW788388 Hydrate (Sigma-Aldrich ref. SML0116), widely used in this type of assay, was used as an inhibition control. Initially, the phosphorylation reaction assay was optimized with serial substrate dilutions (1:8 was estimated to be the best) and different ATP concentrations, with 100 μM being estimated to be the ideal concentration in this case. The concentration of recombinant kinase was also determined by performing assays with serial dilutions of the same (from 20 ng to 0.62 ng), with 3 ng being determined as the ideal concentration per well (final volume 100 μL).

Once the optimal reaction conditions had been determined, the chemically synthesized compounds were tested, initially dissolved in dimethyl sulfoxide (DMSO) at a concentration of 1 mM. From this stock solution, dilutions were made in kinase buffer (Tris-HCl pH 7.5 40 mM; $MgCl_2$ 20 mM; bovine serum albumin (BSA) 0.1 mg/mL) of 10 μM and concentrations of 5 and 10 μM were evaluated. The reactions were carried out in a total volume of 50 μL in Microtiter®

Microlite™ 1+ microtiter plastic plates (96 wells) (Thermo Scientific, ref. 7571), which allow their use for determining luminescence in luminometers.

The reaction included:

| | |
|---|---|
| Inhibitors (10μM ) | 5 μL |
| ATP (1mM) | 5 μL |
| Alk5 kinase (10 ng/uL) | 3 μL |
| Buffer | 37 μL |

Incubation was carried out for 2 hours at room temperature with stirring and in darkness, following the instructions for the commercial system.

After this time, 50 μL of commercial substrate (diluted 1:8) was added, left to stirr for 10 minutes at room temperature, and the luminescence was read using a Luminoskan Ascent instrument (Thermo Scientific, Waltham, USA).

All reactions were performed in duplicate.

**C. TOXICITY STUDIES OF SYNTHETIC CHEMICAL COMPOUNDS IN CULTURED CELLS**

The first study conducted investigated whether these compounds were toxic to human cells in culture. Their effect on macrophages and intestinal epithelial cells was analyzed.

***C.1. Cytotoxicity assay (MTT).***

**To quantify the percentage of cells that remained viable in each of the experiments, THP-1-XBlue™-CD14 macrophages (InvivoGen, 8 to 13 passages), derived from human THP-1 monocytes, which stably express the gene for the alkaline phosphatase enzyme inducible by the NF-kB and AP-1 promoters.**

The MTT dye (3-(4,5-dimethylthiazol-2-yl)-2,5-diphenyltetrazolium bromide) (Biotium) was used. This assay is based on the cleavage of the yellow MTT tetrazolium salt to form purple formazan crystals, produced by metabolically active cells. Formazan is solubilized with DMSO and its concentration is determined by spectrophotometry; the optical density is proportional to the number of viable cells.

First, THP1-XBlue™-CD14 monocytes (InvivoGen, 8 to 13 passages) were differentiated into a macrophage-like phenotype using phorbol-12 -myristate-13-acetate (PMA, Sigma Aldrich), which at 100 ng/mL has been shown to be the most effective alternative for obtaining THP-1 macrophages, similar to peripheral blood mononuclear cells (PBMCs) (Starr et al., 2018). To do this, the cells were seeded in 96-well plates at a density of 3 x 105 cells/mL in RPMI 1640 medium (Gibco) supplemented with 10% FBS, penicillin, streptomycin (both at a concentration of 100 μg/mL, Sigma-Aldrich), and PMA (100 ng/mL) (Sigma-Aldrich), and were cultured at 37 °C, 5% $CO_2$ for 48 h. Subsequently, the medium was replaced with an equivalent one, but without PMA supplementation, and the cells were cultured at 37 °C, 5% $CO_2$ for another 24 h. The cells were then washed twice with PBS (10 mM, pH 7.4), and 100 μL of RPMI 1640 supplemented with penicillin and streptomycin (100 μg/mL) but without FBS was added. They were seeded in 96-well plates under conditions identical to those used in the anti-inflammation assays. The cells were then washed with PBS and treated with different compounds: 1) PBS (10 μL) (positive growth control, (vehicle)); 2) different concentrations of the chemical compounds produced (1 μM, 5 μM, and 10 μM). The plates were incubated for 24 hours at 37 °C, 5% $CO_2$, and then 10 μL of MTT was added to each well and the plate was incubated for 4 hours at 37 °C, 5% $CO_2$. The formazan crystals formed were dissolved in 180 μL of DMSO and the plate was incubated for 20 minutes with shaking at room temperature. The absorbance was measured in each well at 595 nm (problem) and 655 nm (background), following the manufacturer's instructions, using an iMark™ Microplate Absorbance Reader (Bio-Rad).

The samples were analyzed in triplicate. The percentage of viable cells was calculated using the following formula:

$$\% \text{ Viability} = (A_{595} - A_{655} \text{ Problem sample}) / (A_{595} - A_{655} \text{ Positive control}) \times 100$$

**D. DETERMINATION OF ANTI-INFLAMMATORY ACTIVITY IN MACROPHAGES IN CULTURE OF THE SYNTHESIZED COMPOUNDS**

*D.1. Ensayo de efecto antiinflamatorio*

To study the anti-inflammatory activity of the synthesized chemical compounds, we used the transcription factor

NF-kB, which is involved in inflammatory processes, as a biomarker. For this purpose, we used the THP-1-XBlue™-CD14 cell line (InvivoGen), derived from human THP-1 monocytes, which stably expresses the gene for the alkaline phosphatase enzyme inducible by the NF-kB and AP-1 promoters. In addition, these cells stably overexpress CD14, a specific Toll-like receptor (TLR) for macrophages, such as TLR4. Thus, when this CD14 receptor is stimulated (for example, with bacterial lipopolysaccharide, LPS, InvivoGen), THP1-XBlue™-CD14 cells activate these transcription factors and consequently the synthesis and secretion of alkaline phosphatase, which is easily detected by adding the commercial substrate QUANTI-Blue™ (InvivoGen), which turns blue/purple in the presence of secreted phosphatase.

THP1-XBlue™-CD14 cells (InvivoGen, 8 to 13 passages) were processed as mentioned above, differentiating them into macrophages in the presence of PMA (Sigma Aldrich). The cells were then washed twice with PBS (10 mM, pH 7.4), and 100 μL of RPMI 1640 supplemented with penicillin and streptomycin (100 μg/mL) but without FBS was added. Subsequently, the cultured cells were treated with: 1) 10 μL PBS (negative control); 2) GW788388 (positive control); and 5 μM concentrations of the synthesized compounds. After 1 h of incubation, lipopolysaccharide (LPS) from Escherichia coli bacteria (InvivoGen) was added at a final concentration of 300 ng/mL, and the cultures were maintained at 37 °C, 5% $CO_2$ for 24 h.

After this time, 20 μL of supernatant was removed from each well and transferred to a new plate, and 180 μL of QUANTI-Blue™ reagent was added. After 5 h of incubation at 37 °C, the absorbance at 655 nm was measured in the wells of the plate using an iMark™ Microplate Absorbance Reader (Bio-Rad). The samples were analyzed in quadruplicate.

On the other hand, the same process was carried out in parallel, without adding the stimulant LPS to the plates, to check for possible intrinsic inflammatory activity of these compounds.

Eight replicates of the biological tests were performed.

**E. CELL VIABILITY AND CHEMICAL COMPOUND TOXICITY STUDIES 31 (PS1801) AND 26 (PS1906)**

To study whether these compounds from examples 26 and 31 were toxic to human cells in culture, their effect on macrophages and intestinal epithelial cells was analyzed.

***E.1. Quantification of cell viability.***

To quantify the percentage of cells that remained viable in each of the experiments, THP-1-XBlue™-CD14 macrophages (InvivoGen, 8 to 13 passages) were evaluated, derived from human THP-1 monocytes, which stably express the gene for the alkaline phosphatase enzyme inducible by the NF-kB and AP-1 promoters. Human intestinal epithelial Caco-2 cells (ATCC®, HTB-37TM, 10 to 12 passages) were also evaluated. To do this, Trypan blue (Gibco) was used as an exclusion stain. This dye penetrates cells with damaged membranes (a sign of cell death) and stains their cytoplasm dark blue. To do this, the cell suspensions tested were centrifuged at 500 x g (5 min at 24 °C), the cell sediments were resuspended in 0.1 mL of PBS, and then 10 μL were mixed with 10 μL of trypan blue (0.4%) filtered through 0.22 μm. The percentage of live cells was determined in 10 μL of the above mixture using a chamber and a Countess™ cell counter (Invitrogen).

Eight replicates of the biological assays were performed.

***E.2. Cytotoxicity assay (MTT).***

In this second cytotoxicity assessment on THP-1-XBlue-CD14 and Caco-2, the technique previously described was used with the MTT dye (3-(4,5-dimethylthiazol-2-yl)-2,5-diphenyltetrazolium bromide) (Biotium).

To do this, both types of cells were seeded in 96-well plates under conditions identical to those used in the anti-inflammation assays. The cells were then washed with PBS and treated with different compounds: 1) PBS (10 μL) (positive growth control, vehicle); 2) 20% DMSO (negative control, as high doses of this compound are cytotoxic); 3) different concentrations of the compounds PS1801 and PS1906 (1, 5, and 10 μM). The plates were incubated for 24 hours at 37 °C, 5% $CO_2$, and then 10 μL of MTT was added to each well and the plate was incubated for 4 hours at 37 °C, 5% $CO_2$. The formazan crystals formed were dissolved in 180 μL of DMSO, and the plate was incubated for 20 minutes with shaking at room temperature. The absorbance was measured in each well at 595 nm (problem) and 655 nm (background), following the manufacturer's instructions, using an iMark™ Microplate Absorbance Reader (Bio-Rad). The samples were analyzed in triplicate.

Eight replicates of the biological assays were performed.

The percentage of viable cells was also calculated using the following formula:

$$\% \text{ Viability} = (A_{595} - A_{655} \text{ Problem sample}) / (A_{595} - A_{655} \text{ Positive control}) \times 100$$

## F. DETERMINATION OF ANTI-INFLAMMATORY ACTIVITY IN MACROPHAGES IN CULTURE OF COMPOUNDS PS1801 AND PS1906

*F.1. Anti-inflammatory effect test*

To study the anti-inflammatory activity of the chemical compounds PS1801 and PS1906, we followed the previously described protocol using the transcription factor NF-kB as a biomarker, using the THP-1-XBlue™-CD14 cell line (InvivoGen).

Similar to the assays described above, THP1-XBlue™-CD14 monocytes (InvivoGen, 8 to 13 passages) were differentiated to a macrophage-like phenotype using PMA (Sigma Aldrich) and seeded in 96-well plates at a density of 3 x 105 cells/mL in RPMI 1640 medium (Gibco) supplemented with 10% FBS, penicillin, and streptomycin (both at a concentration of 100 μg/mL). mL in RPMI 1640 medium (Gibco) supplemented with 10% FBS, penicillin, streptomycin (both at a concentration of 100 μg/mL, Sigma-Aldrich), and PMA (100 ng/mL) (Sigma-Aldrich), and were cultured at 37 °C, 5% $CO_2$ for 48 h. The medium was then replaced with an equivalent medium, but without PMA supplementation, and the cells were cultured at 37 °C, 5% $CO_2$ for a further 24 h. The cells were then washed twice with PBS (10 mM, pH 7.4), and 100 μL of RPMI 1640 supplemented with penicillin and streptomycin (100 μg/mL) but without FBS was added.

Subsequently, the cells in culture were treated with: 1) 10 μL PBS (negative control); 2) GW788388 (positive control); and 3) 5 μM of the compounds PS1801 and PS1906. After 1 h of incubation, lipopolysaccharide (LPS) from *Escherichia coli* bacteria (InvivoGen) was added at a final concentration of 300 ng/mL, and the cultures were kept at 37 °C, 5% $CO_2$ for 24 h.

After this time, 20 μL of supernatant was extracted from the culture medium of each well and transferred to a new plate, and 180 μL of QUANTI-Blue™ reagent was added. After 5 h of incubation at 37 °C, the absorbance at 655 nm was measured in the wells of the plate using an iMark™ Microplate Absorbance Reader (Bio-Rad). The samples were analyzed in quadruplicate.

On the other hand, the same process was carried out in parallel, without adding the LPS stimulant to the plates, to check for possible intrinsic inflammatory activity of these compounds.

Eight replicates of the biological assays were performed.

## G. STATISTICAL ANALYSIS

The experimental results obtained were subjected to analysis of variance (ANOVA), and for those parameters that did not show a normal population distribution, the nonparametric Kruskal-Wallis test was applied. GraphPad Prism version 7.0 for Windows (GraphPad Software, La Jolla, California, USA, www.graphpad.com) was used. *P value < 0.05, **P value < 0.01, ***P value < 0.001.

## H. EVALUATION OF ANTI-INFLAMMATORY ACTIVITY IN VIVO: MURINE MODEL OF ACUTE LUNG INJURY (ALI)

In order to evaluate the potential anti-inflammatory effect of the PS1906 molecule in an in vivo model, we selected the ALI model induced by tracheal instillation of lipopolysaccharide (LPS), which has been widely described in the literature and previously performed by members of the research group (Clemente-Moragón et al., 2020). This murine model is a model of acute lung inflammation or damage associated with a long-term fibrotic period, so it can be used to study both inflammatory lung diseases and pulmonary fibrotic processes.

*H. 1. Animals*

Mice of the C57BL/6J-OlaHsd strain were chosen for this model. Adult mice were kept in pathogen-free conditions in a temperature-controlled room with a 12-hour light-dark cycle. Food and water were available ad libitum. The mice were anesthetized intraperitoneally with an injection of an anesthetic cocktail containing atropine sulfate (1 mg/kg; B. Braun, 1 mg/mL), xylazine hydrochloride (20 mg/kg; Rompun® Bayer, 20 mg/mL), and ketamine (100 mg/kg; Anesketin Dechra, 100 mg/mL). All experimental and other scientific procedures involving animals complied with EU Directive 2010/63EU and Recommendation 2007/526/EC, enacted into Spanish law under Real Decreto 53/2013. Animal protocols were approved by local ethics committees and the Animal Protection Area of the Autonomous Community of Madrid.

*H.2. Selection of doses and administration of treatments.*

In vitro studies were taken into account when selecting the doses to be administered, with a concentration of 10

μM being considered the maximum effective (non-toxic) concentration. Assuming an approximate blood volume of 2 mL for a mouse weighing about 30 g, and taking into account a loss due to distribution, metabolism, or secretion of 50% of the products, we calculated dose 1 (D1) and selected dose 2 (D2) as twice the first. Thus, taking into account the respective molecular weights, the corresponding doses for the PS1906 product were 156 μg/kg (D1) and 312 (D2). Physiological saline was used as a diluent for the preparation of the different doses. All doses were prepared to administer the same volume in the mouse. The doses were administered intravenously with the animal properly anesthetized moments before the induction of pulmonary inflammation.

*H.3. Induction of the acute pulmonary inflammation model.*

With the animal properly anesthetized, the trachea was exposed, a catheter (Introcan Safety 22G, Braun) was inserted, and the mice received an intratracheal injection of sterile lipopolysaccharide (LPS) (L2262, Sigma) at a high dose of 10 mg/kg in 25 μl of 1X PBS, followed by 200 l of air to ensure deposition in each lung. Healthy control group mice received an intratracheal injection of 25 μl of PBS. After intratracheal administration, the wounds were closed, and the mice were allowed to recover with free access to food and water.

*H.4. Evaluation of inflammatory infiltrate in bronchoalveolar lavage.*

After 24 h, the animals were sacrificed and the lungs were washed with 1.5 mL of cold PBS through a tracheal catheter (Introcan Safety 22G, Braun), and a total of 1 mL of bronchoalveolar lavage fluid was extracted from each mouse for the detection of neutrophils and lymphocytes by flow cytometry. Detection was performed by size and complexity, as well as positive labeling for the Ly6G marker (neutrophils) and the CD4 marker (lymphocytes), as well as the total number of cells using a FACS Canto-3L flow cytometer equipped with DIVA software (BD Biosciences). The data were analyzed with FlowJo software (Ashland).

*H.5. Processing of lungs for histopathological evaluation*

Upon completion of the protocol and prior to euthanizing the animals, the lungs were removed by separating the right lobe from the left lobe. The right lobe was fixed in 4% paraformaldehyde (PFA) for 24 hours at 4°C, and the left lobe was rapidly frozen in liquid nitrogen for subsequent protein isolation and immunoblotting assays. The PFA-fixed right lobes were placed in 70% alcohol at 4°C and cut transversely into two portions. For the histopathological scoring of acute lung injury (ALI score), both transverse portions were dehydrated using a series of ethanol, cleared in xylene, embedded in paraffin wax, cut into 5 μm sections, mounted on Superfrost Plus slides (Thermo Fisher Scientific), and stained with hematoxylin and eosin solution. The tissues were processed in the CNIC Histopathology Unit, and all slides were scanned with a NanoZoomer-2.0-RS digital slide scanner (C110730®, Hamamatsu, Japan) and visualized with NanoZoomer Digital Pathology software (Hamamatsu). ALI was scored using a semi-quantitative system, using a scale of 0 to 4 that combined assessments of inflammatory cell infiltration of the airspace and vessel wall, alveolar congestion, hemorrhage, alveolar wall thickness, and hyaline membrane formation. A score of 0 indicates no injury, 1 indicates a minor injury, 2 a moderate injury, 3 a severe injury, and 4 a very severe injury.

*H.6. Statistical analysis*

The data were transformed to a logarithmic scale to better appreciate the differences between groups. They are presented as mean ± standard deviation and were analyzed using Prism v7 software (Graph pad, Inc.). Comparisons between groups were performed using one-way ANOVA. P values were adjusted using Dunnet's multiple comparison method. *** $p < 0.0001$ vs. control; # $p < 0.05$, # # $p < 0.01$, # # $p < 0.001$ vs. LPS group for n = 4-7 mice.

## RESULTS

## RESULTS OF ALK5 KINASE ACTIVITY INHIBITION ASSAYS

The assays performed showed that several compounds exhibited Alk5 kinase inhibitory activity, compared to the compound GW788388, which exhibited approximately 25% inhibition. The compounds exhibited the inhibitory effects shown in Table 2.

Table 2. Formula and reference of compounds with in vitro Alk5 kinase inhibitory activity.

| Molecular Formula / MW | Reference | % kinase activity reduction |
|---|---|---|
| $C_{18}H_{15}N_3O_2$ 305.337 | 31/ PS1801 | 79.1 |
| $C_{20}H_{16}N_2O_2$ 316.36 | 16 | 75.4 |
| $C_{13}H_{10}N_2O$ 210.236 | 12 | 87.5 |
| $C_{15}H_{10}N_2O$ 234.258 | 26/ PS1906 | 93.4 |

The values for % reduction in kinase activity refer to a 100% reduction in activity of the inhibition control compound GW788388 Hydrate (Sigma-Aldrich ref. SML0116).

Figure 1 shows the results obtained with the different compounds, where it can be seen that compound PS1906 (16) was one of those that showed the greatest inhibitory effect on the tyrosine kinase Alk5.

**RESULTS OF IN VITRO CYTOTOXICITY TESTS ON CELL CULTURES**

As shown in Figure 2, treatments with compounds used at 1 μM have no cytotoxic effects on THP-1 macrophages (Figure 2), as they do not decrease cell viability. Compound 16 was found to be toxic to cells at concentrations of 5 μM and 10 μM, while the rest were not toxic. The compound GW788388 (control), a known inhibitor of Alk5 kinase activity and lacking cytotoxic activity, was also tested (Figure 2).

Eight replicates of the biological tests were performed.

**VIABILITY AND CYTOTOXICITY RESULTS OF COMPOUNDS PS1801 AND PS1906**

As shown in Figure 3, treatments with compounds PS1801 and PS1906 have no cytotoxic effects on Caco-2 epithelial cells (Figure 3A) or THP-1 macrophages (Figure 3B) at all concentrations used, as they do not decrease cell viability. These results confirm previous data obtained in THP-1 macrophages and highlight the absence of toxicity in intestinal epithelial cells. DMSO was used as a cell-toxic substance (positive control), as well as the compound GW788388 (control), a known inhibitor of Alk5 kinase activity and lacking cytotoxic activity.

It was decided to continue the tests using a concentration of 5 μM, as this was the concentration at which cell viability was least reduced in both cell types.

**9.5. PS1801 AND PS1906 COMPOUNDS EXHIBIT ANTI-INFLAMMATORY ACTIVITY IN HUMAN THP-1-XBLUE™ CD14 MACROPHAGES.**

To study the anti-inflammatory activity of compounds PS1801 and PS1906, we used NF-kB, a transcription factor involved in inflammation, as a biomarker. THP-1-XBlue™-CD14 macrophages were stimulated with LPS (Figure 4), a promoter of the expression of this transcription factor, which binds to the TLR4 receptor, overexpressed in these macrophages. At a concentration of 5 μM, the compounds significantly inhibited LPS-dependent NF-kB production ($p < 0.001$). These results confirmed the results obtained previously.

In turn, the activity of these vesicles was tested in a control environment, without LPS stimulation (Figure 5), revealing that on their own they do not produce NF-kB expression in vitro. These results indicate that the compounds PS1906 and PS1801 are useful for reducing inflammation derived from effectors dependent on this transcription factor, with particular interest when used as treatment in a pre-inflammatory environment.

**9.6. RESULTS OBTAINED IN VIVO IN A MURINE MODEL OF INFLAMMATION AND ACUTE LUNG INJURY**

To study anti-inflammatory activity in vivo, we chose a model of acute lung injury (ALI) used to study inflammatory lung diseases such as acute respiratory distress syndrome. This model is evaluated using a score derived from histopathological analysis of fixed lung sections stained with hematoxylin and eosin. Panel A of Figure 6 shows representative images of the lung from each group, where macroscopic differences can be observed. Quantification of the ALI score (shown in panel B of Fig. 6) shows a positive effect for the PS1906 product. Flow cytometry analysis of bronchoalveolar lavage fluid showed a dose-dependent anti-inflammatory effect of the PS1906 compound in both neutrophils (panel C of Fig. 6) and lymphocytes (panel D of Fig. 6; although these results are not statistically significant, probably due to the low participation of this cell type in the early stages of acute lung injury).

**REFERENCES**

Clemente-Moragón A, Gómez M, Villena-Gutiérrez R, Lalama DV, García-Prieto J, Martinez F, Sánchez-Cabo F, Fuster V, Oliver E, Ibáñez B. Metoprolol exerts a non-class effect against ischaemia-reperfusion injury by abrogating exacerbated inflammation. Eur Heart J. 2020 Dec 7;41 (46):4425-4440. https://doi.org/10.1093/eurheartj/ehaa733

**Claims**

1. A compound of formula **I:**

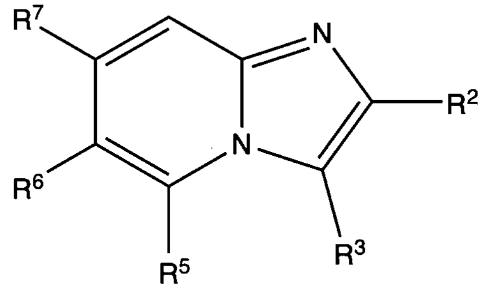

**I**

or a pharmaceutically acceptable salt thereof, wherein:

$R^2$ represents H; an aryl, wherein the aryl group is unsubstituted, or substituted with alkyl groups of 1 to 5 carbon atoms, or substituted with a haloalkyl, or substituted with an alkoxy group of 1 to 5 carbon atoms,
$R^3$ represents H; a halogen; a haloalkyl; an alkoxycarbonyl with 2 to 5 carbon atoms; an amino; an aryl or heteroaryl, where the aryl and heteroaryl groups are unsubstituted or are substituted with alkyl groups with 1 to 5 carbon atoms, or with an alkoxycarbonyl of 2 to 5 carbon atoms;
$R^5$ represents H; an alkenyl of 2 to 10 carbon atoms; an alkoxycarbonyl of 2 to 5 carbon atoms; a nitro group; a carboxyl; an alkyl of 1 to 5 carbon atoms unsubstituted or substituted with one or more hydroxy groups; an N-(3-methylene-2-oxoindolin-5-yl)-3-(piperidin-1-yl)propanamide group); or carbaldehyde,
$R^6$ represents a hydrogen atom (H); an unsubstituted alkyl of 1 to 5 carbon atoms; an alkyl group of 1 to 5 carbon atoms substituted with one or more hydroxy groups; an alkoxy group of 1 to 5 carbon atoms; a halogen selected from F, Cl, I; an alkoxycarbonyl (-O-(C=O)-R) where R has 1 to 3 carbon atoms; a nitro grup; an alkenyl of 3 to 10 carbon atoms unsubstituted or substituted; an alkenyl of 2 carbon atoms substituted with a phenyl (styryl); an aryl, wherein the aryl group is unsubstituted or substituted by one or more hydroxyl groups, or substituted with one or more methoxy groups; heteroaryl; or heteroaryl wherein the heteroaryl group is selected from dibenzothiophenyl, a group of formula II, a group of formula **III:**

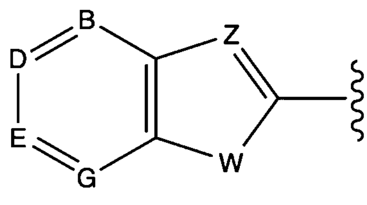

**II**

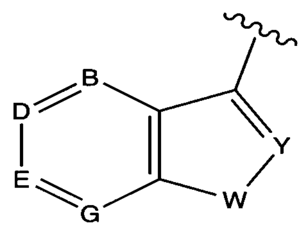

**III**

where D, B, E, G independently represent N, CH, or C-R, where R is unsubstituted alkyl of one to three carbon atoms, unsubstituted aryl, or unsubstituted cycloalkyl,
Z represents CH or N,
Y represents CH or N,
W represents O, S, or NH,

$R^7$ represents a hydrogen atom (H); an unsubstituted aryl group or an aryl group substituted with one or more hydroxy groups, or substituted with one or more methoxy groups; a halogen; an alkenyl of 3 to 10 C atoms, unsubstituted or substituted by an aryl group; an alkenyl of 2 carbon atoms substituted with a phenyl (styryl); an alkoxycarbonyl of 2 to 5 carbon atoms; a heteroaryl group; or heteroaryl where the heteroaryl group is selected from benzothiophenyl, benzofuranyl, benzopyrrolyl; dibenzothiophenyl, a group of formula II and a group of formula III as defined above,

- wherein:

  - at least 3 of the substituents $R^2$, $R^3$, $R^5$, $R^6$, and $R^7$ are simultaneously H, and the position of carbon 8 of the imidazo[1,2a]pyridine structure has no substituents, and
  - when $R^5$ is a carbaldehyde, at least one of $R^2$, $R^3$, $R^6$ or $R^7$ is other than H,
  - when $R^6$ is an aryl or substituted aryl, $R^3$ is not a substituted aryl
  - when $R^3$ represents an amino group, $R^6$ does not represent hydrogen
  - when $R^2$ represents an unsubstituted aryl, or an aryl substituted with a methyl or methoxy group, at least one of the substituents $R^2$, $R^3$, $R^5$, $R^6$, and $R^7$ is not H
  - at least one of the groups $R^3$, $R^5$, $R^6$, $R^7$ is different from H, when $R^2$ is an unsubstituted aryl, or is an aryl substituted with a halogen, a methyl or a methoxy.

**2.** A compound according to claim 1, wherein $R^2$ represents an aryl substituted with a $CF_3$ group or with an alkoxy group of 1 to 5 carbon atoms; and preferably, in addition, $R^3$ and $R^5$ are simultaneously H.

**3.** A compound according to claim 1, wherein:

- $R^2$ represents an aryl substituted with alkoxy of 1 to 5 carbon atoms, and
- simultaneously $R^6$ is:
  - an alkyl of one to 5 carbon atoms, or
  - an aryl group substituted with a hydroxyl, and preferably, in addition, $R^3$ and $R^5$ are simultaneously H.

**4.** A compound according to claim 1, wherein $R^2$ represents a group selected from aryl substituted with alkoxy of 1 to 5 carbon atoms; and simultaneously $R^6$ is

- alkyl of one to 5 carbon atoms, or
- an aryl group substituted with a hydroxyl;

and preferably $R^3$ and $R^5$ are simultaneously H.

**5.** A compound according to claim 1, wherein $R^2$ represents a phenyl substituted with an alkoxy of 1 to 5 carbon atoms or phenyl substituted with trihalomethyl; and

- simultaneously $R^7$ is a heteroaryl group, preferably 2-benzo[b]furyl;

and more preferably, $R^3$, $R^4$, $R^5$, and $R^6$ are simultaneously H.

**6.** A compound according to claim 1, wherein:

- $R^2$ represents aryl substituted with alkoxy of 1 to 5 carbon atoms and $R^7$ is aryl substituted with hydroxyl or with an alkoxy group of 1 to 5 carbon atoms, or
- $R^2$ represents aryl substituted with alkoxy of 1 to 5 carbon atoms and $R^7$ represents alkenyl substituted with an aryl group;

and preferably, in addition, $R^3$ and $R^5$ are simultaneously H.

**7.** A compound according to claim 1, wherein $R^2$ represents an aryl substituted with an alkoxy of 1 to 5 carbon atoms and $R^7$ is an aryl substituted with a hydroxyl or with an alkoxy group of 1 to 5 carbon atoms or an alkenyl substituted with an aryl group; and preferably, in addition, $R^3$ and $R^5$ are simultaneously H.

**8.** A compound according to claim 1, wherein $R^2$ represents an aryl substituted with an alkoxy of 1 to 5 carbon atoms, $R^3$ is H, $R^6$ is H, an alkyl group or an aryl group, and $R^7$ is H or an aryl substituted with a hydroxyl or with an an alkoxy group of 1 to 5 carbon atoms; and preferably $R^3$ and $R^5$ are simultaneously H.

**9.** A compound according to claim 1, wherein $R^3$ is an unsubstituted aryl; and preferably, further, $R^2$, $R^5$, and $R^6$ are simultaneously H; and more preferably, further, $R^2$, $R^5$, $R^6$, and $R^7$ are simultaneously H.

**10.** A compound according to claim 1, wherein $R^3$ represents an unsubstituted aromatic heterocycle, or an aromatic heterocycle substituted with an alkoxycarbonyl group having 2 to 5 carbon atoms; and preferably, in addition, $R^2$, $R^5$, and $R^6$ are simultaneously H; and more preferably, in addition, $R^2$, $R^5$, $R^6$, and $R^7$ are simultaneously H.

**11.** A compound according to claim 1, wherein $R^3$ is a group selected from benzofuranyl, benzothiophenyl, and indole, unsubstituted or substituted with an alkoxycarbonyl group of 2 to 5 carbon atoms; and preferably, furthermore, $R^2$, $R^5$, and $R^6$ are simultaneously H; and more preferably, furthermore, $R^2$, $R^5$, $R^6$, and $R^7$ are simultaneously H.

**12.** A compound according to claim 1, wherein $R^3$ is a group selected from benzofuranyl, benzothiophenyl, and indole, substituted or substituted with an alkoxycarbonyl group of 2 to 5 carbon atoms; and simultaneously, $R^7$ is a group selected from benzofuranyl, benzothiophenyl, and indole; preferably, $R^2$, $R^5$, and $R^6$ are simultaneously H.

**13.** A compound according to claim 1, wherein $R^5$ represents H, an ethoxycarbonyl, a nitro group, a carboxyl, a hydroxymethyl, a carbaldehyde, or an ethoxyl, and preferably, $R^2$, $R^3$, $R^6$, and $R^7$ are simultaneously H.

**14.** A compound according to claim 1, wherein $R^6$ or $R^7$ represents a fragment

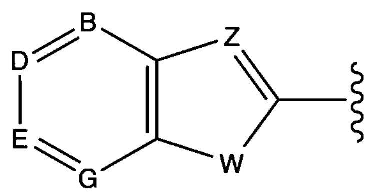

**II**

wherein D, B, E, G independently represent CH or C-R, wherein R may be unsubstituted alkyl, unsubstituted aryl, or unsubstituted cycloalkyl,
Z represents CH, and
W represents O.

**15.** A compound according to claim 1, wherein $R^6$ or $R^7$ represents a fragment

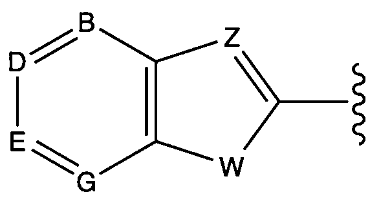

**II**

wherein D, B, E, and G independently represent CH, Z represents C, and W represents O.

**16.** A compound according to claim 1, wherein $R^6$ represents an alkyl group of 1 to 5 carbon atoms or an alkoxycarbonyl (-O-(C=O)-R) where R has 1 to 3 carbon atoms, and preferably, in addition, $R^2$, $R^3$, $R^5$, and $R^7$ are simultaneously H.

**17.** A compound according to claim 1, wherein $R^7$ represents an unsubstituted aryl group, and preferably, furthermore, $R^2$,

$R^3$, $R^5$, and $R^7$ are simultaneously H.

**18.** A compound according to claim 1 selected from:

- Imidazo[1,2-a]pyridine-5-carboxylic acid (2)
- Ethyl imidazo[1,2-a]pyridine-5-carboxylate (3)
- 3-Phenylimidazo[1,2-a]pyridine (4)
- Imidazo[1,2-a]pyridin-5-ylmethanol (5)
- 2-(4-Methoxyphenyl)-6-methylimidazo[1,2-a]pyridine (8)
- 4-(Imidazo[1,2-a]pyridin-7-yl)phenol (12)
- 4-(2-(4-(Trifluoromethyl)phenyl)imidazo[1,2-a]pyridin-7-yl)phenol (13)
- 4-(2-(4-(Trifluoromethyl)phenyl)imidazo[1,2-a]pyridin-6-yl)phenol (15)
- 4-(2-(4-Methoxyphenyl)imidazo[1,2-a]pyridin-7-yl)phenol (16)
- 4-(2-(4-Methoxyphenyl)imidazo[1,2-a]pyridin-6-yl)phenol (17)
- (E)-7-Styrylimidazo[1,2-a]pyridine (19)
- (E)-2-(4-Methoxyphenyl)-7-styrylimidazo[1,2-a]pyridine (20)
- 7-(3,4-Dimethoxyphenyl)imidazo[1,2-a]pyridine (21)
- 6-Iodo-2-(4-(trifluoromethyl)phenyl)imidazo[1,2-a]pyridine (25)
- 7-(Benzofuran-2-yl)imidazo[1,2-a]pyridine (26) PS1906
- 4-(Imidazo[1,2-a]pyridin-6-yl)phenol (27)
- (E)-6-Styrylimidazo[1,2-a]pyridine (28)
- 6-(Benzofuran-2-yl)imidazo[1,2-a]pyridine (29)
- 6-(3,4-Dimethoxyphenyl)imidazo[1,2-a]pyridine (30)
- Ethyl 5-(imidazo[1,2-a]pyridin-3-yl)-1H-indole-2-carboxylate (31) (PS1801)
- 7-(Benzofuran-3-yl)imidazo[1,2-a]pyridine (32)
- 6-(Benzofuran-3-yl)imidazo[1,2-a]pyridine (33)
- 7-(Furan-3-yl)imidazo[1,2-a]pyridine (34)
- 6-(Furan-3-yl)imidazo[1,2-a]pyridine (35)
- 7-(Thiophen-3-yl)imidazo[1,2-a]pyridine (36)
- 6-(Thiophen-3-yl)imidazo[1,2-a]pyridine (37)
- 7-(Pyridin-4-yl)imidazo[1,2-a]pyridine (38)
- 6-(Pyridin-4-yl)imidazo[1,2-a]pyridine (39)
- 7-(Dibenzothiophen-3-yl)imidazo[1,2-a]pyridine (40)
- 6-(Dibenzothiophene-3-yl)imidazo[1,2-a]pyridine (41)
- Ethyl 5-(7-(benzofuran-2-yl)-3-imidazo[1,2-a]pyridin)-1H-indole-2-carboxylate, (42)
- 2-(4-Methoxyphenyl)-6-iodo-imidazo[1,2-a]pyridine,
- N-(3-(imidazo[1,2-a]pyridin-5-ylmethylen)-2-oxoindolin-5-yl)-3-(piperidin-1-yl)propanamide.

**19.** A compound according to claim 1, wherein the compound of formula I is selected from

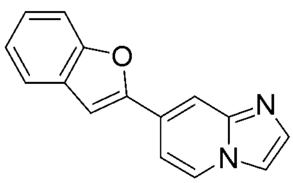

and

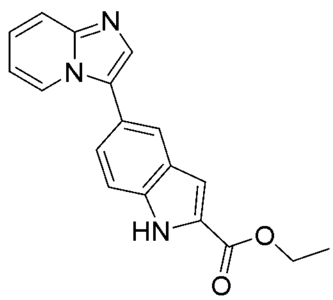

**20.** A compound of formula I, defined in any of claims 1 to 17, for use as a medicament.

**21.** The compound of formula I, according to the preceding claim, for use in treating an inflammatory disease in a subject.

**22.** The compound of formula I, according to the previous claim, wherein the inflammatory disease is an inflammatory lung disease selected from acute respiratory distress syndrome, chronic obstructive pulmonary disease, pulmonary fibrosis, pulmonary hypertension, pulmonary inflammation, preferably acute respiratory distress syndrome and

pulmonary fibrosis.

**23.** The compound of formula I according to any of the preceding claims 20 to 22, wherein the compound is ethyl 5-(imidazo[1,2-a]pyridin-3-yl)-1H-indole-2-carboxylate.

**24.** The compound of formula I according to any of the preceding claims 20 to 22, wherein the compound is 7-(benzofuran-2-yl)imidazo[1,2-a]pyridine.

**25.** The compound of formula I, according to any of the preceding claims 20 to 22, in a concentration between 15 mg/kg and 10 μg/kg, more preferably between 1 mg/kg and 50 μg/kg, and most preferably between 800 and 80 μg/kg.

**26.** A pharmaceutical composition comprising one or more compounds of formula I, defined in any of claims 1 to 19, and

one or more additional active compounds, and/or
one or more pharmaceutically acceptable carriers, adjuvants, or vehicles.

**27.** The pharmaceutical composition according to the preceding claim, wherein one or more pharmaceutically acceptable carriers are selected from a disintegrant, surfactant, binder, lubricant, and combinations thereof.

**28.** The pharmaceutical composition according to one of the preceding claims 26 to 27, wherein one or more additional active compounds are selected from steroids, corticosteroids, and nonsteroidal anti-inflammatory drugs.

**29.** The pharmaceutical composition according to one of the preceding claims 26 to 28, wherein the one or more additional active compounds is one or more compounds of formula I defined in any of claims 1 to 18.

**30.** A pharmaceutical composition, defined in any of preceding 25 to 29 for its use as a medicament.

**31.** A pharmaceutical composition according to the preceding claim for use in the treatment of an inflammatory disease, preferably an inflammatory lung disease selected from acute respiratory distress syndrome, chronic obstructive pulmonary disease, pulmonary fibrosis, pulmonary hypertension, pulmonary inflammation, preferably acute respiratory distress syndrome and pulmonary fibrosis.

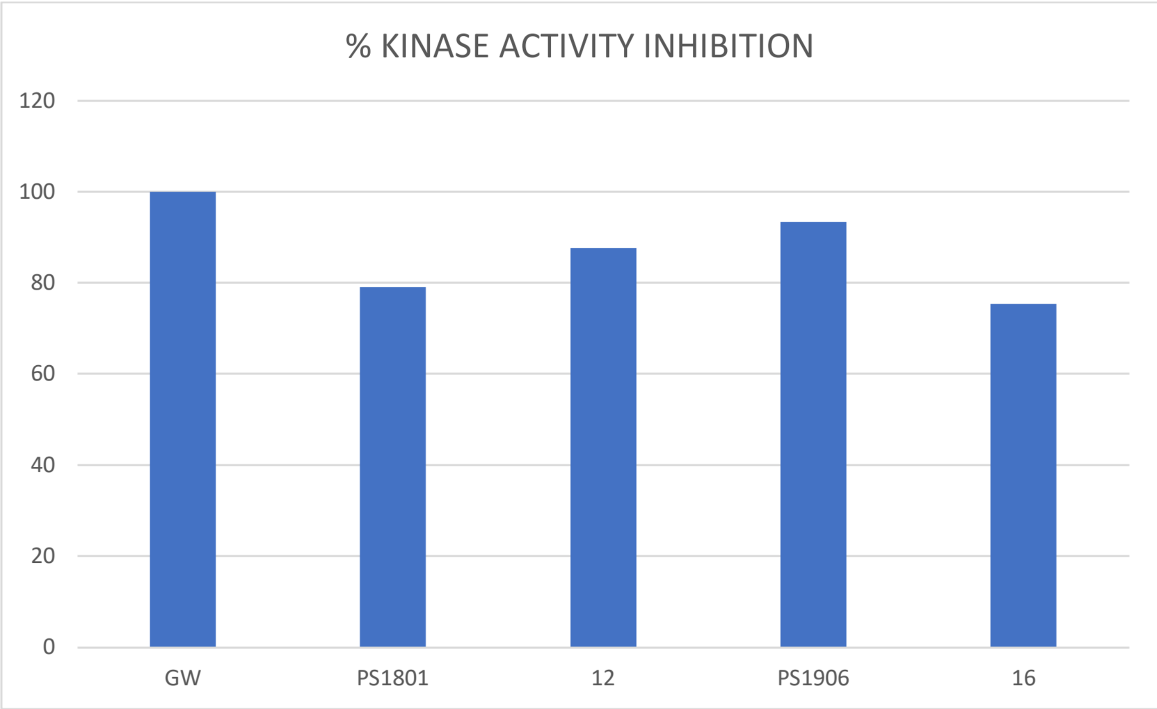


Figure 1

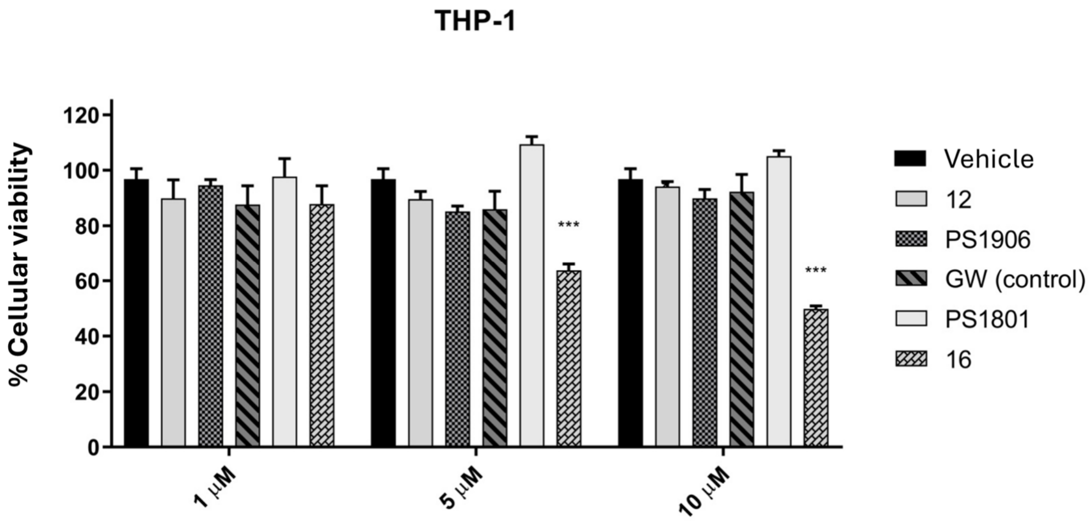


Figure 2

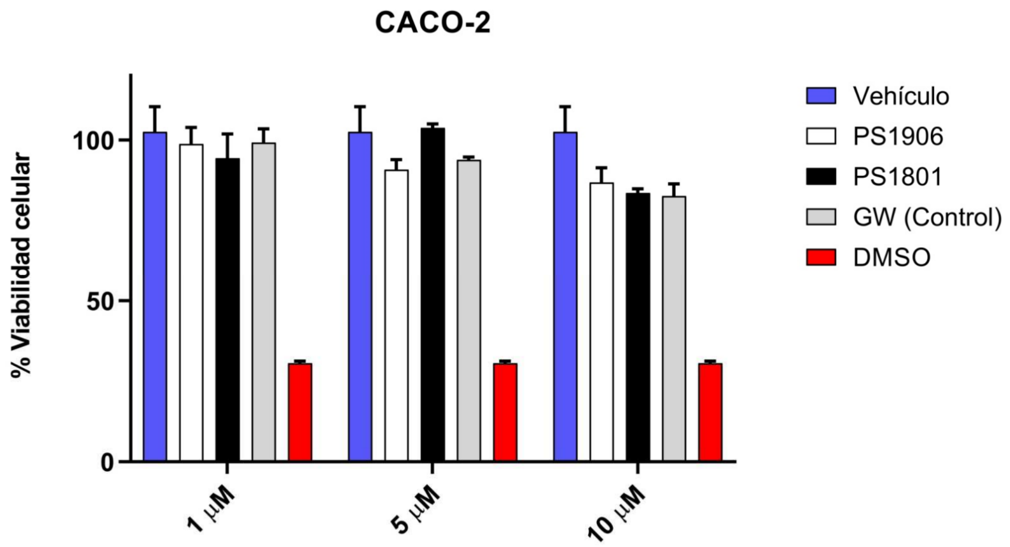
CACO-2
% Viabilidad celular
100
50
0
1 μM
5 μM
10 μM
Vehículo
PS1906
PS1801
GW (Control)
DMSO

t

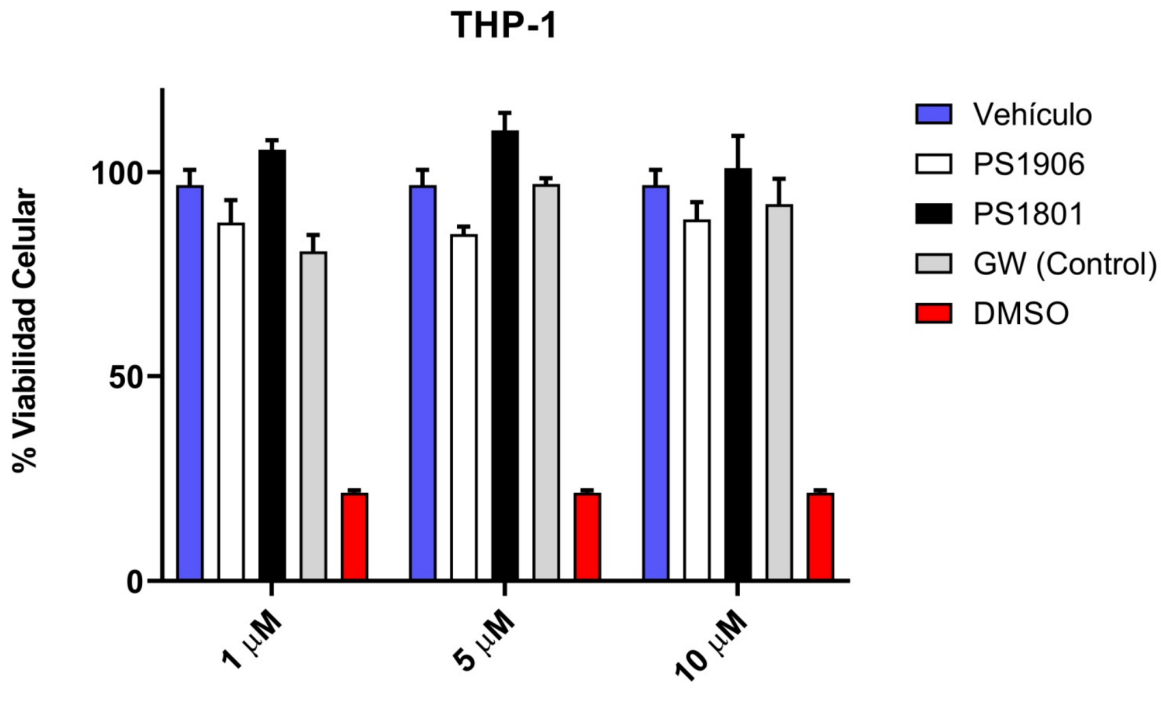
THP-1
% Viabilidad Celular
100
50
0
1 μM
5 μM
10 μM
Vehículo
PS1906
PS1801
GW (Control)
DMSO

Figure 3

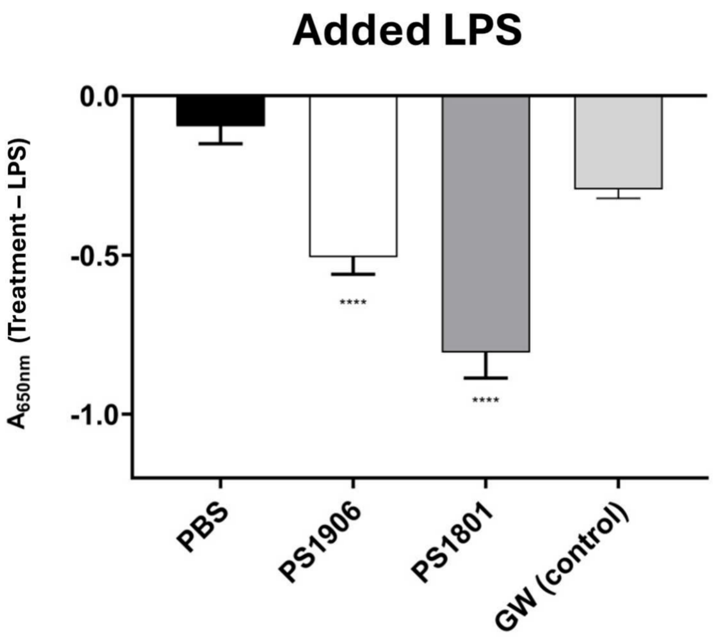


Figure 4

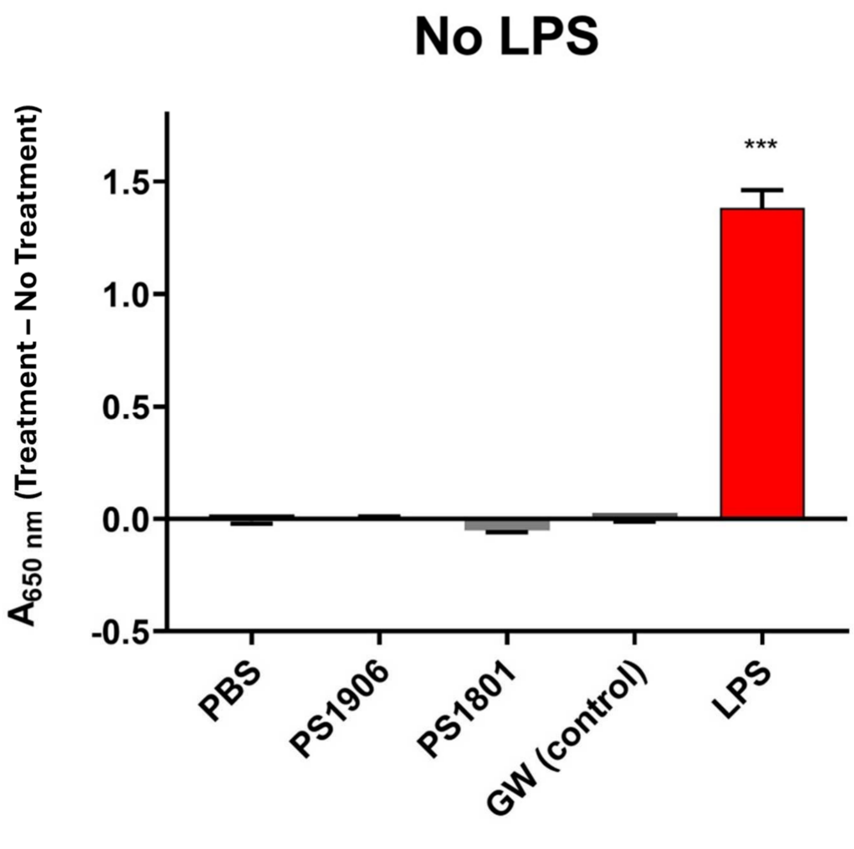


Figure 5

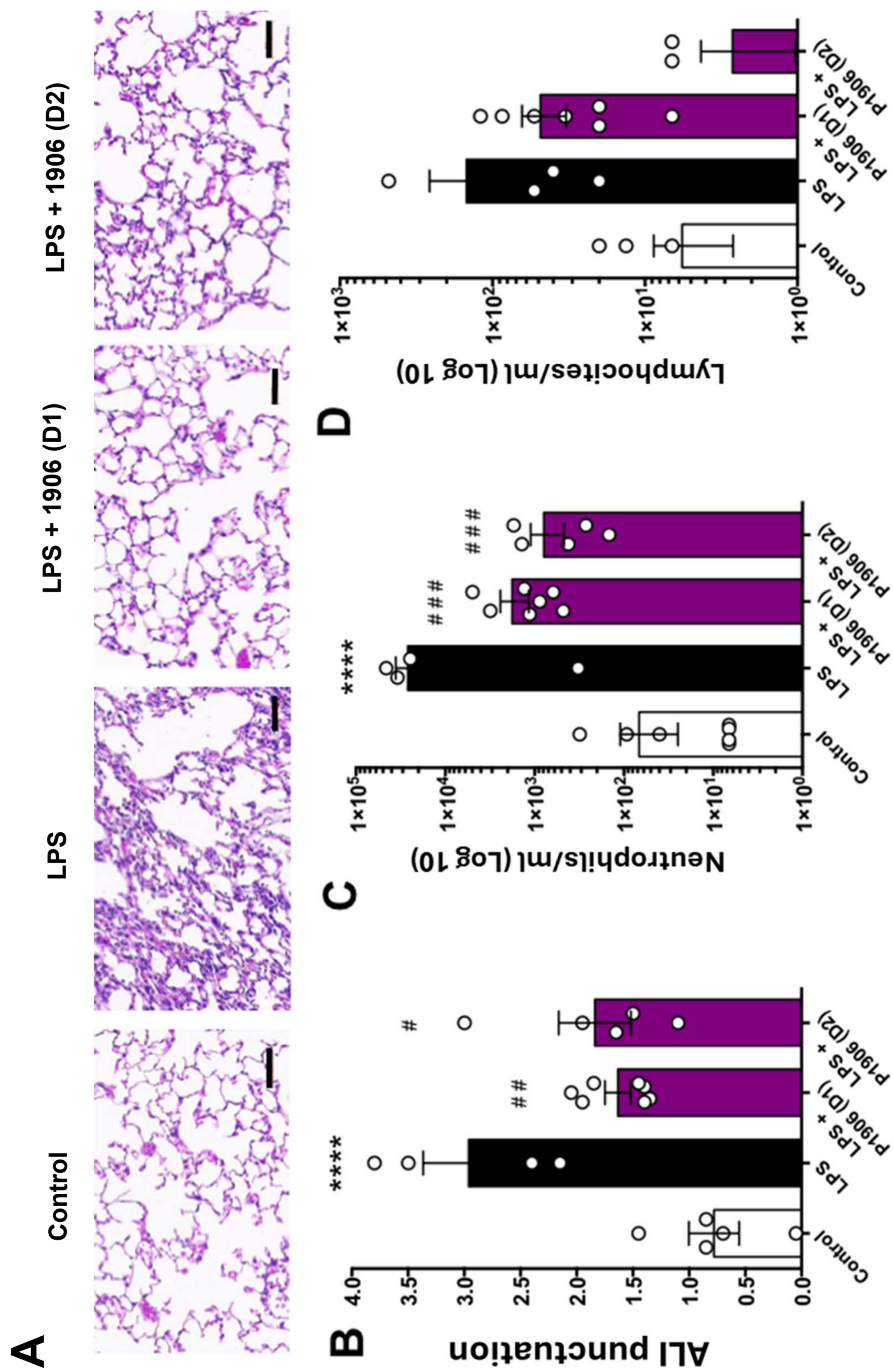
A
Control
LPS
LPS + 1906 (D1)
LPS + 1906 (D2)
B
ALI punctuation
C
Neutrophils/ml (Log 10)
D
Lymphocites/ml (Log 10)
Control
LPS
LPS + P1906 (D1)
LPS + P1906 (D2)

Figure 6

## INTERNATIONAL SEARCH REPORT

International application No.
PCT/ES2024/070403

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
C07D, A61K, A61P

Documentation searched other than minimum documentation to the extent that such documents are included in the fields searched

Electronic data base consulted during the international search (name of data base and, where practicable, search terms used)

EPODOC, INVENES, WPI, TXT-DB, NPL, XPESP, CAS

C. DOCUMENTS CONSIDERED TO BE RELEVANT

| Category* | Citation of document, with indication, where appropriate, of the relevant passages | Relevant to claim No. |
|---|---|---|
| X | WO 2013147711 A1 (AGENCY SCIENCE TECH & RES) 03/10/2013, claims 1-13; page 61, lines 26-32. | 1-17, 20-22, 25-31 |
| X | WO 2019099336 A1 (BRISTOL MYERS SQUIBB CO) 23/05/2019, pages 2, 3, 70-80 and 103-130; claim 1. | 1-17, 20-22, 25-31 |
| A | US 2004122044 A1 (MAUL CORINNA ET AL.) 24/06/2004, paragraphs 2, 3, 8-17 and 60-63; claim 1. | 1-31 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 25/09/2024 | **(04/10/2024)** |
| Name and mailing address of the ISA/<br>OFICINA ESPAÑOLA DE PATENTES Y MARCAS Paseo de la Castellana, 75 - 28071 Madrid (España)<br>Facsimile No.: 91 349 53 04 | Authorized officer<br>N. Martín Laso<br>Telephone No. 913493278 |

Form PCT/ISA/210 (second sheet) (July 2022)

## INTERNATIONAL SEARCH REPORT

International application No.
PCT/ES2024/070403

C (continuation). DOCUMENTS CONSIDERED TO BE RELEVANT

| Category * | Citation of documents, with indication, where appropriate, of the relevant passages | Relevant to claim No. |
|---|---|---|
| A | RODRIGUEZ, J. C. et al. "Microwave-assisted synthesis and luminescent activity of imidazo[1,2-a]pyridine derivatives". Journal Of Heterocyclic Chemistry, 2020, Vol. 57, Nº 5, pages 1-9, <DOI: 10.1002/jhet.3950>. See abstract; table 5. | 1-31 |
| A | WO 2008014219 A2 (SMITHKLINE BEECHAM CORP ET AL.) 31/01/2008, pages 39-41, schemes1-7; claim 1. | 1-31 |

Form PCT/ISA/210 (continuation of second sheet) (July 2022)

**INTERNATIONAL SEARCH REPORT**

International application No.

PCT/ES2024/070403

CLASSIFICATION OF SUBJECT MATTER

***C07D471/04*** (2006.01)
***A61K31/437*** (2006.01)
***A61P29/00*** (2006.01)

Form PCT/ISA/210 (extra sheet) (July 2022)

## INTERNATIONAL SEARCH REPORT

Information on patent family members

International application No.
PCT/ES2024/070403

| Patent document cited in the search report | Publication date | Patent family member(s) | Publication date |
|---|---|---|---|
| WO2013147711 A1 | 03.10.2013 | BR112014024255A2 | 20.06.2017 |
| | | BR112014024255B1 | 28.12.2021 |
| | | PL2831079T T3 | 31.01.2018 |
| | | US2019315755 A1 | 17.10.2019 |
| | | US11040978 B2 | 22.06.2021 |
| | | US2019211020 A1 | 11.07.2019 |
| | | NO2831079T T3 | 13.01.2018 |
| | | JP2019031560 A | 28.02.2019 |
| | | JP6662984B B2 | 11.03.2020 |
| | | US2018208600 A1 | 26.07.2018 |
| | | ES2647959T T3 | 27.12.2017 |
| | | DK2831079T T3 | 27.11.2017 |
| | | SG10201706594P A | 28.09.2017 |
| | | AU2017219039 A1 | 14.09.2017 |
| | | EP3321268 A1 | 16.05.2018 |
| | | SG11201406207P A | 27.11.2014 |
| | | JP2015511638 A | 20.04.2015 |
| | | JP6475158B B2 | 27.02.2019 |
| | | CN104350055 A | 11.02.2015 |
| | | CN104350055B B | 31.05.2019 |
| | | KR20150002639 A | 07.01.2015 |
| | | KR102153320B B1 | 08.09.2020 |
| | | US2015038506 A1 | 05.02.2015 |
| | | US10280168 B2 | 07.05.2019 |
| | | US2014371199 A1 | 18.12.2014 |
| | | US9908886 B2 | 06.03.2018 |
| | | AU2013240612 A1 | 16.10.2014 |
| | | AU2013240612B B2 | 25.05.2017 |
| | | EP2831079 A1 | 04.02.2015 |
| | | EP2831079 A4 | 18.11.2015 |
| WO2019099336 A1 | 23.05.2019 | ES2945435T T3 | 03.07.2023 |
| | | US2023098244 A1 | 30.03.2023 |
| | | US11739098 B2 | 29.08.2023 |
| | | US2021253593 A1 | 19.08.2021 |
| | | JP2021503004 A | 04.02.2021 |
| | | JP7265554B B2 | 26.04.2023 |
| | | KR20200086709 A | 17.07.2020 |
| | | CN111448190 A | 24.07.2020 |
| | | CN111448190B B | 26.09.2023 |
| | | EP3710440 A1 | 23.09.2020 |
| | | EP3710440 B1 | 05.04.2023 |
| US2004122044 A1 | 24.06.2004 | HUP0400877 A2 | 30.08.2004 |
| | | PL366437 A1 | 24.01.2005 |
| | | MXPA03008894 A | 08.12.2003 |
| | | JP2004531512 A | 14.10.2004 |
| | | EP1372644 A2 | 02.01.2004 |
| | | WO02080911 A2 | 17.10.2002 |
| | | WO02080911 A3 | 25.09.2003 |

Form PCT/ISA/210 (patent family annex) (July 2022)

## INTERNATIONAL SEARCH REPORT

Information on patent family members

International application No.

PCT/ES2024/070403

| Patent document cited in the search report | Publication date | Patent family member(s) | Publication date |
|---|---|---|---|
| | | DE10117184 A1 | 17.10.2002 |
| | | CA2442986 A1 | 17.10.2002 |
| WO2008014219 A2 | 31.01.2008 | JP2009544723 A | 17.12.2009 |
| | | US2009215818 A1 | 27.08.2009 |
| | | EP2046333 A2 | 15.04.2009 |
| | | EP2046333 A4 | 15.09.2010 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013147711 A
- WO 2008014219 A
- US 2004122044 A
- WO 2019099336 A


### Non-patent literature cited in the description


- **LING LE** ; **LEE WC**. Tgf-beta type I receptor (Alk5) kinase inhibitors in oncology. *Curr Pharm Biotechnol*, December 2011, vol. 12 (12), 2190-202, https://doi.org/10.2174/138920111798808257
- **RODRIGUEZ, J. C. et al.** Microwave-assisted synthesis and luminescent activity of imidazo[1,2a] pyridine derivatives. *Journal of Heterocyclic Chemistry*, 2020
- **CHEN W**. A potential treatment of COVID-19 with TGF-β blockade. *Int J Biol Sci.*, 21 April 2020, vol. 16 (11), 1954-1955
- **FERREIRA-GOMES M** ; **KRUGLOV A** ; **DUREK P** ; **HEINRICH F** ; **TIZIAN C** ; **HEINZ GA** ; **PASCUAL-REGUANT A** ; **DU W** ; **MOTHES R** ; **FAN C**. SARS-CoV-2 in severe COVID-19 induces a TGF-β-dominated chronic immune response that does not target itself. *Nat Commun*, 30 March 2021, vol. 12 (1), 1961, https://doi.org/10.1038/s41467-021-22210-3
- **SHEN WX** ; **LUO RC** ; **WANG JQ** ; **CHEN ZS**. Features of Cytokine Storm Identified by Distinguishing Clinical Manifestations in COVID-19. *Front Public Health*, 24 May 2021, vol. 9, 671788, https://doi.org/10.3389/fpubh.2021.671788
- **MÁRQUEZ-FLORES YK** ; **CAMPOS-ALDRETE ME** ; **SAGADO-ZAMORA H** ; **CORREA-BASURTO J** ; **MELÉNDEZ-CAMARGO ME**. Acute and chronic anti-inflammatory evaluation of imidazo[1,2-A]pyridine carboxylic acid derivatives and docking analysis. *Medicinal Chemistry Research*, 2012, 3491-3498, https://doi.org/10.1007/s00044-011-9870-3
- **BUDUMURU P** ; **GOLAGANI S** ; **PUSHPANJALI B**. Microwave-assisted synthesis of imidazo[1,2-a]pyridine derivatives and their anti-inflammatory activity. *IJPSR*, 2019, vol. 10 (3), 1172-1179, http://dx.doi.org/10.13040/IJPSR.0975-8232
- **ENGERS DW** ; **BOLLINGER SR** ; **FELTS AS** ; **VADUKOOT AK** ; **WILLIAMS CH** ; **BLOBAUM AL** ; **LINDSLEY CW** ; **HONG CC** ; **HOPKINS CR**. Discovery, synthesis and characterization of a series of 7-aryl-imidazo[1,2-a]pyridine-3-ylquinolines as activin-like kinase (ALK) inhibitors.. *Bioorg Med Chem Lett.*, 15 September 2020, vol. 30 (18), 127418, https://doi.org/10.1016/j.bmcl.2020.127418
- **NOLTE M** ; **MARGADANT C**. Controlling Immunity and Inflammation through Integrin-Dependent Regulation of TGF-β. *Trends Cell Biol.*, January 2020, vol. 30 (1), 49-59, https://doi.org/10.1016/j.tcb.2019.10.002
- **CLEMENTE-MORAGÓN A** ; **GÓMEZ M** ; **VILLENA-GUTIÉRREZ R** ; **LALAMA DV** ; **GARCÍA-PRIETO J** ; **MARTINEZ F** ; **SÁNCHEZ-CABO F** ; **FUSTER V** ; **OLIVER E**. Ibáñez B. Metoprolol exerts a non-class effect against ischaemia-reperfusion injury by abrogating exacerbated inflammation.. *Eur Heart J.*, 07 December 2020, vol. 41 (46), 4425-4440, https://doi.org/10.1093/eurheartj/ehaa733